# EUROPÄISCHE PATENTSCHRIFT

(11) **EP 0 934 081 B1**
(45) Veröffentlichungstag und Bekanntmachung des Hinweises auf die Patenterteilung: **09.06.2004**
(21) Anmeldenummer: 97943750.6
(22) Anmeldetag: 09.09.1997
(51) Int. Cl.: A61K 47/48, A61K 35/00, A61P 35/00

(54) **ANTINEOPLASTISCH WIRKENDE ALBUMINKONJUGATE**
ANTINEOPLASTIC CONJUGATES OF ALBUMIN
CONJUGUES D'ALBUMINE A ACTION ANTINEOPLASTIQUE

(30) Priorität: 11.09.1996 DE 19636889
(43) Veröffentlichungstag der Anmeldung: 11.08.1999
(73) Patentinhaber: Kratz, Felix, 79232 March (DE)
(72) Erfinder: Kratz, Felix, 79232 March (DE)
(74) Vertreter: Perrey, Ralf, Dr. Dipl.-Chem.
(86) Internationale Anmeldenummer: PCT/DE1997/002000
(87) Internationale Veröffentlichungsnummer: WO 1998/010794

(56) Entgegenhaltungen:
- EP-A- 0 359 347
- EP-A- 0 495 265
- EP-A- 0 745 390
- WO-A-88/00837
- WO-A-91/08220
- WO-A-92/02255
- DE-A- 4 122 210
- BEYER ET AL.: "SYNTHESIS OF TRANSFERRIN AND ALBUMIN CONJUGATES OF THE ANTICANCER DRUGS CHLORAMBUCIL, DOXORUBICIN AND DAUNORUBICIN" EUR. J. PHARM. SCIENCES, Bd. 4, Nr. Suppl., 1996, Seite S111 XP002062901
- NATHAN ET AL.: "Strategies for Covalent Attachment of Doxorubicin to poly(PEG-Lys), a New Water-Soluble Poly(ether urethane)" J. BIOACT. COMPAT. POLYM., Bd. 9, Nr. 3, 1994, Seiten 239-251, XP002062902
- FUJIWARA K ET AL: "THE USE OF N-(AMINOBENZOYLOXY)SUCCINIMIDE AS A TWO-LEVEL HETEROBIFUNCTIONAL AGENT FOR THE PREPARATION OF HAPTEN-PROTEIN CONJUGATES. DAUNOMYCIN AS A MODEL HAPTEN WITH AN AMINO GROUP" JOURNAL OF IMMUNOLOGICAL METHODS, Bd. 134, Nr. 2, 1990, Seiten 227-235, XP000673599
- SEZAKI ET AL.: "Macromolecule-drug conjugates intargeted cancer chemotherapy" CRC CRIT. REV. THER. DRUG CARRIER SYST. , Bd. 1, Nr. 1, 1984, Seiten 1-38, XP002062903
- CALICETI P ET AL: "PREPARATION AND PROPERTIES OF MONOMETHOXY POLY(ETHYLENE GLYCOL) DOXORUBICIN CONJUGATES LINKED BY AN AMINO ACID OR A PEPTIDE AS SPACER" FARMACO, Bd. 48, Nr. 7, 1.Juli 1993, Seiten 919-932, XP000371926
- SENTER P D ET AL: "POLY(ETHYLENE GLYCOL)-DOXURUBICIN CONJUGATES CONTAINING -LACTAMASE-SENSITIVE LINKERS" BIOCONJUGATE CHEMISTRY, Bd. 6, Nr. 4, 1.Juli 1995, Seiten 389-394, XP000517227
- LEE ET AL.: "Synthesis of Drug-Macromolecule Conjugates: Conjugates of 5-Fluorouracil to Human Serum Albumin and Poly-Lysine" YAKHAK HOECHI, Bd. 33, Nr. 5, 1989, Seiten 267-272, XP002062904
- DEMANT ET AL.: "Characterization of the cooperative cross-linking of doxorubicin N-hydroxysuccinimide ester derivatives to water soluble proteins" BIOCHIM. BIOPHYS. ACTA, Bd. 1118, Nr. 1, 1991, Seiten 83-90, XP002062905

## Beschreibung

Diese Erfindung betrifft tumorhemmende Proteinkonjugate bestehend aus einem geeigneten Trägersystem und zytostatischen Verbindungen. Weiterhin betrifft die Erfindung Verfahren zu der Herstellung von solchen Konjugaten und die Verwendung dieser.

Immunokonjugate bzw. Proteinkonjugate sind Verbindungen, die aus einer geeigneten Trägersubstanz, wie z.B. einem Antikörper, einem Wachstumsfaktor, einer hormon- bzw. peptidähnlichen Struktur, oder einem Protein, und einem oder mehreren zytotoxischen Wirkstoffen, wie zB. Zytostatika, Toxinen oder radioaktiven Isotopen, bestehen. Die Trägersubstanzen besitzen i.d.R. die Eigenschaft, sich bevorzugt im Tumorgewebe anzureichern, so daß auf diesem Wege ebenso der an die Trägersubstanz gebundene Wirkstoff im Tumorgewebe angereichert und somit eine selektivere antitumorale Therapie erzielt wird. Chemoimmunokonjugate bezeichnen Konjugate von Trägersubstanzen und zytostatischen Verbindungen, wobei der Träger i.d.R. ein Antikörper ist.

Die zur Zeit klinisch eingesetzten Zytostatika gegen Krebserkrankungen besitzen eine Reihe von schweren systemischen Nebenwirkungen und weisen keine Anreicherung im Tumorgewebe auf, so daß nach neuen Derivaten bzw. Formulierungen geforscht wird, die eine selektivere antitumorale Therapie ermöglichen. Zu diesem Zwecke werden Chemoimmunokonjugate bzw. Protein- oder Polymerkonjugate bestehend aus einer geeigneten Trägersubstanz und Zytostatika entwickelt.

Als Trägersubstanzen kommen unter anderem Antikörper, Wachstumsfaktoren, Serumproteine, hormon- bzw. peptidähnliche Strukturen oder Polymere in Frage, für die i.d.R. eine Anreicherung im Tumorgewebe bekannt ist (Mägerstädt, M.: Antibody Conjugates and Malignant Disease, Library of Congress 1990; Chadwick, C.M.:Receptors in Tumour Biology, Cambridge University Press, 1984, Seymour, L.W. *CRC Crit. Rev. Ther. Drug Carrier Sys*. (1992), 9, 135-187; Maeda, H.; Matsumura, Y. *CRC Crit. Rev. Ther. Drug Carrier Sys.* (1989), *6*, 193-210).

Die vorliegende Erfindung umfaßt die Trägerproteine humanes Serumtransferrin und Serumalbumin, deren Anreicherung im Tumorgewebe in der Literatur dokumentiert ist (Ward, S.G. Taylor, R.C.: 1-54, in Metal-Based Drugs (Gielen, M.F. (Ed.)). Freund Publishing House Ltd, 1988; Sinn, H., Schrenk. H.H., Friedrich, A., Schilling, U. und Maier-Borst, W. (1990), *Nucl. Med.Biol. Vol. 17(8),* 819-827) sowie Polyethylenglykole (PEGs) als Träger von zytostatischen Verbindungen (Topchieva, I.N. (1990), *Polym.Sci.USSR*, *32*, 833-851; *Poly(ethylene glycol) Chemistry: Biotechnical and Biomedical Applications* (1992), Ed. J.M. Harris, Plenum Press, New York).

PEGs aufgrund ihrer Bioverträglichkeit, ihrer guten Wasserlöslichkeit und synthetischen Divergenz für die Entwicklung von therapeutischen Polymerkonjugaten sehr geeignet. In den letzten Jahren sind PEGs vor allem mit medizinisch bedeutsamen Proteinen und Enzymen konjugiert worden (Übersicht in Topchieva, I.N. *(1990), Polym.Sci.USSR, 32*, 833-851). Die Herstellung von Chemoimmunokonjugaten bzw. Protein- oder Polymerkonjugaten erfolgt grundsätzlich entweder durch direkte Kopplung von Trägersubstanz und Wirkstoff oder mit Hilfe von Spacergruppen, sog. homo- bzw. heterobifunktionelle Reagenzien. Vorwiegend ist bisher das Verfahren der direkten Kopplung angewandt worden, welches jedoch häufig zu polymeren Produkten und nicht eindeutig definierten Konjugaten führt. Kürzlich sind einige Chemoimmunokonjugate (Europäische Patentanmeldung, EP 91-117535 911615, Europäische Patentanmeldung, EP 90-109268 900516, PCT Internationale Patentanmeldung, WO 90-CA251 900809, Britische(UK) Patentanmeldung, GB 83-5104 830224 und Europäische Patentanmeldung, EP 89-102370 890210), die mittels bestimmter bifunktioneller Reagenzien hergestellt wurden, als zytostatisch wirkende Mittel vorgeschlagen worden. Des weiteren sind aus DE 41 22 210 A1 Konjugate tumoraktiver Verbindungen mit Transferrin oder Albumin bekannt, wobei die tumoraktive Verbindung mit N-Hydroxysuccinimid und Carbodiimid aktiviert wird und das so erhaltene Gemisch direkt an das Trägerprotein gekoppelt wird.

Garnett et al. (1983) *Int. J. Cancer,* 31, 661 - 670 beschreibt die Herstellung und Eigenschaften von Arzneistoff-Träger-Antikörper-Präparaten. Insbesondere wird die kovalente Kopplung von Methotrexat an HSA als Träger sowie die weitere Kopplung dieses Konstrukts an einen monoklonalen Antikörper beschrieben.

Es wurde nun gefunden, daß Albumin- bestehend aus Albumin und mindestens einer durch Maleinimid- oder N-Hydroxysuccinimidverbindungen derivatisierten zytostatischen Verbindung, eine der zytostatischen Verbindung gleichwertige oder höhere tumorhemmende Wirksamkeit ausweisen.

Für die Herstellung dieser Protein konjugate sind zytostatische Verbindungen geeignet, wie die Anthrazykline Doxorubicin, Daunorubicin, Epirubicin, Idarubicin und Mitoxandron, die Alkylantien Chlorambucil und Melphalan, die Antimetabolite Methotrexat, 5-Fluorouracil, 5'-Desoxy-5-fluorouridin und Thioguanin, die Taxoide Paclitaxel und Docetaxel, die Camptothecine Topotecan und 9-Aminocamptothecin, die Podophyllotoxinderivate Etoposid, Teniposid und Mitopodozid, die Vinca-Alkaloide Vinblastin, Vincristin, Vindesin und Vinorelbin und eine Verbindung der allgemeinen Formel I, II, III oder IV: 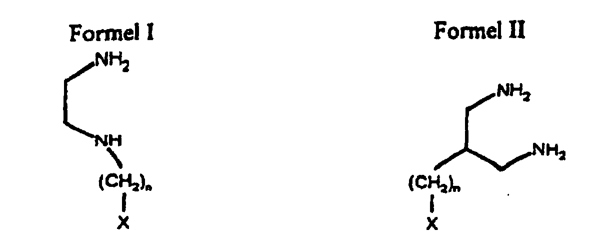 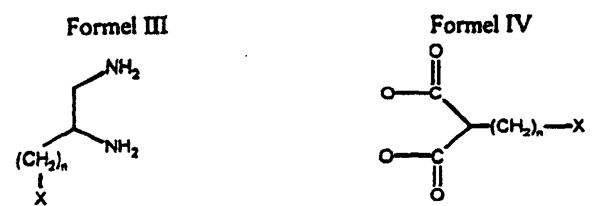 n = 0-6, X = -NH₂, -OH, -COOH, -O-CO-R-COR*, -NH-CO-R-COR*, worin R eine aliphatische Kohlenstoffkette mit 1-6 Kohlenstoffatomen oder eine substituierte bzw. unsubstituierte Phenylengruppe und R* H, Phenyl, Alkyl mit 1-6 Kohlenstoffatomen ist, und die Aminfunktionen mit einer Schutzgruppe wie der *tert*.-Butyloxycarbonyl-Schutzgruppe versehen sind,
die mit einer Maleinimid- oder N-Hydroxysuccinimidverbindung derivatisiert wurden. Die zytostatischen Verbindungen werden dabei i.d.R. mit einer Maleinimidverbindung oder N-Hydroxysuccinimidverbindung umgesetzt, die mindestens eine für die Bindung zum Zytostatikum geeignete funktionelle Gruppe besitzt, wie eine Amino-, Hydroxy-, Carbonsäure-, Carbonsäurechlorid-, Sulfonsäure-, Sulfonsäurechlorid-, Säurehydrazid- oder Hydrazino-, Oxycarbonylchlorid-, Aldehyd- oder Ketogruppe, so daß Maleinimidderivate bzw. N-Hydroxysuccinimidester-Derivate von zytostatischen Verbindungen bereitgestellt werden, wobei die chemische Verknüpfung zwischen Maleinimidverbindung und zytostatischer Verbindung durch eine Amid-, Ester-, Imin-, Hydrazon-, Carboxylhydrazon-, Oxycarbonyl-, Acetal-, oder Ketalbindung erfolgt. Bei den aus den Verbindungen der allgemeinen Formel 1-IV erhaltenen Maleinimid- bzw. N-Hydroxysuccinimidverbindung wird die zytostatische *cis*konfigurierte Platineinheit anschließend eingeführt, d.h. die entsprechenden Platin(II)-Komplexe werden entweder nach Entfernen der Schutzgruppe durch Umsetzen mit einem Tetrachloroplatinat-Salz oder mit *cis*-[PtA₂B] (A = Halogen, B = (NH₃)₂, Ethylendiamin, Propandiamin, 1,2-Diaminocyclohexan) erhalten.

Durch Umsetzen der derivatisierten zytostatischen Verbindungen mit nativem oder thioliertem Albumin werden Proteinkonjugate bereitgestellt, die einfach und effektiv hergestellt werden, die eine hohe Reinheit besitzen, die eine im Vergleich zu mehreren der ursprünglichen zytostatischen Verbindungen ausgezeichnete Wasserlöslichkeit besitzen, die in physiologischem Puffer stabile Formulierungen sind und die eine *in vitro* antiproliferative Wirksamkeit gegen menschliche Tumorzellen aufweisen, die denen der ungebundenen Zytostatika gleichwertig oder überlegen ist. Des weiteren zeigen die Konjugate *in vivo* eine gleichwertige bzw. verbesserte antitumorale Wirksamkeit und eine verbesserte Verträglichkeit. Die durch solche Kopplungen realisierten Protein konjugate, die für eine selektivere Behandlung von Krebserkrankungen sehr geeignet sind, sind Gegenstand der Erfindung und werden im folgenden beschrieben.

Das Verfahren zur Synthese der Proteinkonjugate erfolgt bei den Konjugaten mit Maleinimidderivaten in vier Schritten (Schritt 1 bis 4), bei den Konjugaten mit N-Hydroxysuccinimidester-Derivaten in drei Schritten (Schritt 1, 2 und 4):
- Schritt **1:**: Synthese von Maleinimid- bzw. N-Hydroxysuccinimidverbindungen
- Schritt 2:: Synthese von Maleinimidderivaten bzw. N-Hydroxysuccinimidester-Derivaten zytostatischer Verbindungen
- Schritt 3:: Thiolierung des Trägerproteins
- Schritt 4:: Kopplung der in Schritt 2 erhaltenen zytostatischen Verbindung an natives oder thioliertes Trägerprotein

### Schritt 1: Synthese von Maleinimid- bzw. N-Hydroxysuccinimidverbindungen

Die Maleinimidverbindungen werden im allgemeinen nach einem der zwei folgenden Verfahren hergestellt:

Beim ersten Verfahren wird Maleinsäureanhydrid mit einer aliphatischen Aminoverbindung H₂N-R-Y, wobei R eine aliphatische C-Kette mit 1-6 Kohlenstoffatomen oder eine substituierte bzw. unsubstituierte Benzylgruppe und Y = -OH, -COOH, -SO₃H, -CH(OC₂H₅)₂, R*-C=O, R* = Phenyl oder Alkylrest mit 1 bis 6 Kohlenstoffatomen ist, zur entsprechenden Maleaminsäure und anschließend mit einem bis zwei Äquivalenten Triethylamin (Et₃N) in wasserfreiem Toluol unter azeotroper Entfernung des entstehenden Wassers zur entsprechenden Maleinimidverbindung umgesetzt. Die weitere Derivatisierung der Gruppe Y erfolgt durch Umsetzen der -COOH- bzw. -SO₃H-Gruppe mit Oxalylchlorid oder Thionylchlorid zu den entsprechenden Säurechloriden, durch Umsetzen der Hydroxygruppe mit Bis-(trichlormethyl)-carbonat zu den entsprechenden Oxycarbonylchloriden, durch Umsetzen der Acetalgruppe -CH(OC₂H₅)₂ zu den entsprechenden Aldehyden mit Hilfe von säurekatalytischer Spaltung, wie z.B. durch p-Toluolsulfonsäure, Schwefelsäure oder saures Kieselgel, und durch Umsetzen der Säurechloride mit N-(tert.-Butoxycarbonyl)-alkoholamin bzw. N-(tert.-Butoxycarbonyl)-alkoholhydrazin und anschließender Spaltung mit Trifluoressigsäure oder Chlorwasserstoff (HCl) in Ether, Tetrahydrofuran (THF) oder Dioxan zu den entsprechenden Amino- bzw. Hydrazinoverbindungen.

Beim zweiten Verfahren wird Maleinsäureanhydrid mit einer aromatischen Aminoverbindung H₂N-R-Y, wobei R eine substituierte bzw. unsubstituierte Phenylengruppe und Y = -OH, -COOH, -SO₃H, R*-C=O, R* = Phenyl oder Alkylrest mit 1 bis 6 Kohlenstoffatomen ist, zur entsprechenden Maleaminsäure und anschließend mit Essigsäureanhydrid und wasserfreiem Natriumacetat zur entsprechenden Maleinimidverbindung umgesetzt. Die weitere Derivatisierung der Gruppe Y erfolgt durch Umsetzen der -COOH- bzw. -SO₃H-Gruppe mit Oxalylchlorid oder mit Thionylchlorid zu den entsprechenden Säurechloriden, durch Umsetzen der Hydroxygruppe mit Bis-(trichlormethyl)-carbonat zu den entsprechenden Oxycarbonylchloriden, durch Umsetzen der Säurechloride zu den entsprechenden Aldehyden mit Hilfe von LiA1[OC(CH₃)₃]₃H in THF, durch Umsetzen der Säurechloride in THF oder Ethylacetat mit t-Butylcarbazat und anschließender Spaltung mit Trifluoressigsäure oder HCl in Ether, THF oder Dioxan zu den entsprechenden Säurehydraziden und durch Umsetzen der Säurechloride mit N-(tert.-Butoxycarbonyl)-alkoholamin bzw. N-(tert.-Butoxycarbonyl)-alkoholhydrazin und anschließender Spaltung mit Trifluoressigsäure oder HCl in Ether, THF oder Dioxan zu den entsprechenden Amino- bzw. Hydrazinoverbindungen.

Die Maleinimidverbindungen der allgemeinen Formel VI und VII werden hergestellt, indem man die aus den oben genannten Verfahren erhaltenen Maleinimidverbindungen, bei denen Y -CO-NHNH₂ oder -COR* mit n = 1-6 und R* = H, Phenyl, Alkyl mit 1-6 Kohlenstoffatomen ist, mit Carbonylverbindungen der allgemeinen Formel O=CR*-R-Y oder mit Säurehydraziden der allgemeinen Formel Y-R-R*CO-NH-NH₂ in wasserfreiem THF, Methanol oder Ethanol unter eventuellem Zusatz von p-Toluolsulfonsäure oder Trifluoressigsäure umsetzt. Die weitere Derivatisierung der Gruppe Y erfolgt durch Umsetzen der COOH- bzw. SO₃H-Gruppe mit Oxalylchlorid oder mit Thionylchlorid zu den entsprechenden Säurechloriden, durch Umsetzen der Hydroxygruppe mit Bis-(trichlormethyl)-carbonat zu den entsprechenden Oxycarbonylchloriden.

Die Bismaleinimidverbindungen der allgemeinen Formel XI, wobei zwei Maleinimidverbindungen durch eine Gruppe Z verbunden sind, die eine Diaminoalkan-, Dihydroxyalkan-, Dihydrazinoalkan- oder Carbonsäuredihydrazid-Verbindung darstellt, so daß zwei Maleinimidverbindungen über zwei Amid-, Ester-, Imin-, Hydrazon oder Carboxylhydrazonbindungen miteinander verbunden sind, werden aus den oben erläuterten Maleinimidverbindungen hergestellt, wobei die Synthese der durch Amidbindungen verknüpften Verbindungen durch Umsetzen der Säurechloride der Maleinimidverbindungen mit Diaminoalkanverbindungen NH₂-(CH₂)ₙ-NH₂, n = 2-12, in THF oder Ethylacetat unter eventuellem Zusatz von Et₃N erfolgt, die Synthese der durch eine Esterbindung verknüpften Verbindungen durch Umsetzen der Säurechloride der Maleinimidverbindungen mit Dihydroxyverbindungen HO-(CH₂)ₙ-OH-, n = 2-12, in THF oder Ethylacetat unter eventuellem Zusatz von Et₃N erfolgt, die Synthese der durch eine Iminbindung verknüpften Verbindungen durch Umsetzen der Aldehyde oder Ketone der Maleinimidverbindungen mit Diaminoalkanverbindungen MH₂-(CH₂)ₙ-NH₂, n = 2-12, in wasserfreiem THF, Methanol oder Ethanol unter Zusatz von p-Toluolsulfonsäure oder Trifluoressigsäure erfolgt und die Synthese der durch eine Hydrazon- bzw. Carboxylhydrazonbindung verknüpften Verbindungen durch Umsetzen der Aldehyde oder Ketone der Maleinimidverbindungen mit Dihydrazinoalkanverbindungen NH₂-NH-(CH₂)ₙ-NH-NH₂ bzw. Carbonsäuredihydraziden H₂N-NH-CO-(CH₂)ₙ-CO-NH-NH₂, n = 2-12, in wasserfreiem THF, Methanol oder Ethanol unter Zusatz von p-Toluolsulfonsäure oder Trifluoressigsäure erfolgt.

Die N-Hydroxysuccinimidverbindungen werden im allgemeinen hergestellt, indem N-Hydroxysuccinimid mit Y-R-COOH oder mit Y-R-COCI, wobei R eine substituierte bzw. unsubstituierte Phenylengruppe bedeutet, Y = -OH, -NH₂, -COO-(CH₂)ₙ-OH, -CONH-(CH₂)ₙ-NHBOC, -NHNHBOC, -COO-(CH₂)ₙ-NHNHBOC, -SO₃H, -SO₂-NHNHBOC, -CHO, -COR*, -CO-NHNHBOC mit n = 1-6 und R* = H, Phenyl, Alkyl mit 1-6 Kohlenstoffatomen und BOC die t*ert*.-Butyloxycarbonyl-Schutzgruppe ist, zur entsprechenden N-Hydroxysuccinimidester-Verbindung umgesetzt wird. Dabei wird die Umsetzung ausgehend von Y-R-COOH in einem wasserfreien Lösungsmittel, vorzugsweise Dichlormethan, Acetonitril oder THF unter Zusatz von Dimethylaminopyridin (DMAP) und einem Kondensationsmittel, i.d.R. N,N'-Dicyclohexylcarbodiimid (DCC) oder N-Cyclohexyl-N'-(2-morpholinoethyl)-carbodiimid Metho-p-toluolsulfonat (CMC), zu den entsprechenden Succinimidester-Derivaten durchgeführt. Wird das Säurechlorid Y-R-COCI, das durch Chlorierung mit Säurehalogenierungsreagenzien, wie z.B. mit Oxalylchlorid oder Thionylchlorid erhalten wird, eingesetzt, dann erfolgt die Umsetzung mit N-Hydroxysuccinimid vorzugsweise in wasserfreiem THF, Acetonitril oder Ethylacetat. Die BOC-Schutzgruppe kann im Anschluß mit Trifluoressigsäure oder HCl in Ether oder Dioxan entfernt werden, so daß die entsprechenden Amino- oder Hydrazinoverbindungen sowie die Säurehydrazide als Trifluoracetate oder Hydrochloride erhalten werden. Eine weitere Derivatisierung der Gruppe Y erfolgt durch Umsetzen der Hydroxygruppe mit Bis-(trichlormethyl)-carbonat zu den entsprechenden Oxycarbonylchloriden. N-Hydroxysuccinimidverbindungen der allgemeinen Formel IX und X werden hergestellt, indem man die aus den oben genannten Verfahren erhaltenen N-Hydroxysuccinimidverbindungen, bei denen Y -CO-NHNH₂ oder -COR* mit n = 1-6 und R* = H, Phenyl, Alkyl mit 1-6 Kohlenstoffatomen ist, mit Carbonylverbindungen der allgemeinen Formel O=CR*-R-Y oder mit Säurehydraziden der allgemeinen Formel Y-R-R*CO-NH-NH₂ in wasserfreiem THF, Methanol oder Ethanol unter eventuellem Zusatz von p-Toluolsulfonsäure oder Trifluoressigsäure umsetzt. Die weitere Derivatisierung der Gruppe Y erfolgt durch Umsetzen der -COOH- bzw. -SO₃H-Gruppe mit Oxalylchlorid oder mit Thionylchlorid zu den entsprechenden Säurechloriden, durch Umsetzen der Hydroxygruppe mit Bis-(trichlormethyl)-carbonat zu den entsprechenden Oxycarbonylchloriden.

Die Isolierung der oben genannten Maleinimid- und N-Hydroxysuccinimidester-Verbindungen erfolgt entweder durch Kristallisation, durch Kieselgelsäulenchromatographie oder durch präparative HPLC bzw. LPLC auf Diol-Säule, wie in den untenstehenden Beispielen beschrieben.

### schritt 2: Maleinimidderivate bzw. N-Hydroxysuccinimidester-Derivate zytostatischer Verbindungen

Für die Umsetzung mit den aus Schritt 1 erhaltenen Maleinimid- und N-Hydroxysuccinimidverbindungen sind die in den Ansprüchen 1 bis 3 genannten zytostatischen Verbindungen geeignet. Diese zytostatischen Verbindungen werden mit den im 1. Schritt erläuterten Maleinimid- bzw. N-Hydroxysuccinimidester-Verbindungen umgesetzt, so daß Maleinimidderivate bzw. N-Hydroxysuccinimidester-Derivate von zytostatischen Verbindungen bereitgestellt werden, wobei die chemische Verknüpfung zwischen Maleinimidverbindung bzw. N-Hydroxysuccinimidester-Verbindung und zytostatischer Verbindung durch eine Amid-, Ester-, Imin-, Hydrazon-, Carboxylhydrazon-, Acetal- oder Ketalbindung erfolgt.

Bei den Anthrazyklinen Doxorubicin, Daunorubicin, Epirubicin oder Idarubicin erfolgt die Synthese im einzelnen durch Umsetzen mit den unter Schritt 1 aufgeführten Säuren oder Säurechloriden der Maleinimid- bzw. N-Hydroxysuccinimidverbindungen der Formel V bis X zu den entsprechenden Anthrazyklin-Maleinimidderivaten bzw. Anthrazyklin-Hydroxysuccinimidderivaten in einem Lösungsmittel, vorzugsweise Dimethylformamid (DMF) oder THF unter eventuellem Zusatz einer tertiären Base, i.d.R. Et₃N, oder unter eventuellem Zusatz von DMAP und einem Kondensationsmittel, i.d.R. DCC oder CMC, wobei die Kopplung über die 3'-NH₂-Gruppe des Aminozuckers des Anthrazyklins als Amidbindung erfolgt,
oder durch Umsetzen mit den unter Schritt 1 aufgeführten Aldehyden bzw. Ketonen der Maleinimid- bzw. N-Hydroxysuccinimidverbindungen in einem Lösungsmittel, vorzugsweise DMF, Methanol oder Ethanol, unter eventuellem Zusatz einer Säure, i.d.R. p-Toluolsulfonsäure bzw. Trifluoressigsäure, wobei die Kopplung über die 3'-NH₂-Gruppe des Aminozuckers des Anthrazyklins als Iminbindung erfolgt,
oder durch Umsetzen mit den mit den unter Schritt 1 aufgeführten Aminen der Maleinimid- bzw. N-Hydroxysuccinimidverbindungen in einem Lösungsmittel, vorzugsweise DMF, Methanol oder Ethanol, unter Zusatz einer Säure, i.d.R. p-Toluolsulfonsäure bzw. Trifluoressigsäure, wobei die Kopplung über die C₁₃-Ketoposition des Anthrazyklins als Iminbindung erfolgt, oder durch Umsetzen mit den unter Schritt 1 aufgeführten Säurehydraziden der Maleinimid- bzw. N-Hydroxysuccinimidverbindungen in einem Lösungsmittel, vorzugsweise DMF, Methanol oder Ethanol, unter Zusatz einer Säure, i.d.R. p-Toluolsulfonsäure bzw. Trifluoressigsäure, wobei die Kopplung über die C₁₃-Ketoposition des Anthrazyklins als Carboxyl- bzw. Sulfonylhydrazonbindung erfolgt.

Bei Mitoxandron erfolgt die Synthese im einzelnen durch Umsetzen mit den unter Schritt 1 aufgeführten Säuren oder Säurechloriden der Maleinimid- bzw. N-Hydroxysuccinimidverbindungen der Formel V bis X zu den entsprechenden Mitoxandron-Maleinimidderivaten bzw. Mitoxandron-Hydroxysuccinimidderivaten in einem Lösungsmittel, vorzugsweise DMF oder THF unter eventuellem Zusatz einer tertiären Base, i.d.R. Et₃N, oder unter eventuellem Zusatz von DMAP und einem Kondensationsmittel, i.d.R. DCC oder CMC, wobei die Kopplung über mindestens eine der aliphatischen HO-Gruppen des Mitoxandrons als Esterbindung erfolgt,
oder durch Umsetzen mit den unter Schritt 1 aufgeführten Aldehyden oder Ketonen der Maleinimid- bzw. N-Hydroxysuccinimidverbindungen in einem Lösungsmittel, vorzugsweise THF, Methanol oder Ethanol, unter Zusatz einer Säure, i.d.R. Trifluoressigsäure oder p-Toluolsulfonsäure, wobei die Kopplung über mindestens eine der aliphatischen HO-Gruppen des Mitoxandrons als Acetal- bzw. Ketalbindung erfolgt.

Bei den Alkylantien Chlorambucil und Melphalan erfolgt die Synthese im einzelnen durch Umsetzen von Chlorambucil bzw. Melphalan mit den unter Schritt 1 aufgeführten Hydroxyverbindungen von Maleinimid- bzw. N-Hydroxysuccinimidverbindungen in einem Lösungsmittel, vorzugsweise DMF, Dichlormethan oder THF unter Zusatz von DMAP und einem Kondensationsmittel, i.d.R. DCC oder CMC, zu den entsprechenden Chlorambucil- oder Melphalan-Maleinimidderivaten bzw. Chlorambucil- oder Melphalan-Hydroxysuccinimidverbindungen, wobei die Kopplung über die COOH-Gruppe von Chlorambucil bzw. Melphalan als Esterbindung erfolgt,
oder durch Umsetzen von Chlorambucil bzw. Melphalan mit Säurehalogenierungsreagenzien, wie Oxalylchlorid oder Thionylchlorid, zu den entsprechenden Säurechloriden und anschließendem Umsetzen der Säurechloride in THF oder Ethylacetat mit t-Alkylcarbazaten, i.d.R. tert.-Butylcarbazat, oder unter eventuellem Zusatz einer tertiären Base, i.d.R. Et₃N, oder durch Umsetzen von Chlorambucil bzw. Melphalan in DMF, THF oder Ethylacetat mit t-Alkylcarbazaten, i.d.R. t-Butylcarbazat, unter eventuellem Zusatz von DMAP und einem Kondensationsmittel, i.d.R. DCC oder CMC, und anschließender Spaltung der so erhaltenen Produkte mit Säuren, i.d.R Trifluoressigsäure oder HCl in Ether, THF oder Dioxan, zu den entsprechenden Säurehydraziden von Chlorambucil bzw. Melphalan, die nun wiederum mit einem der in Schritt 1 aufgeführten Aldehyden oder Ketonen der Maleinimid- bzw. N-Hydroxysuccinimidverbindungen in einem Lösungsmittel, vorzugsweise DMF, Methanol oder Ethanol, unter Zusatz einer Säure, i.d.R. Trifluoressigsäure oder p-Toluolsulfonsäure, zu den entsprechenden Maleinimid- bzw. N-Hydroxysuccinimidcarboxylhydrazon-Derivaten von Chlorambucil bzw. Melphalan umgesetzt werden.

Bei 5-Fluorouracil erfolgt die Synthese im einzelnen durch Umsetzen mit den unter Schritt 1 aufgeführten Säurechloriden der Maleinimid- bzw. N-Hydroxysuccinimidverbindungen zu den entsprechenden Mateinimid- bzw. N-Hydroxysuccinimidderivaten von 5-Fluorouracil in einem Lösungsmittel, vorzugsweise THF, unter eventuellem Zusatz einer tertiären Base, i.d.R. Et₃N, wobei die Kopplung über die ¹N- oder ³N-Position von 5-Fluorouracil als Säureamidbindung erfolgt,
oder durch Umsetzen mit den unter Schritt 1 aufgeführten Oxycarbonylchloriden der maleinimid- bzw. N-Hydroxysuccinimidverbindungen zu den entsprechenden Maleinimid- bzw. N-Hydroxysuccinimidderivaten von 5-Fluorouracil in einem Lösungsmittel, vorzugsweise THF unter eventuellem Zusatz einer tertiären Base, i.d.R. Et₃N, wobei die Kopplung über die ¹N-oder ³N-Position von 5-Fluorouracil als Oxycarbonylbindung erfolgt,
oder durch Umsetzen von 5-Fluorouracil mit Formaldehyd und den unter Schritt 1 aufgeführten Carbonsäuren oder Sulfonsäuren zu den entsprechenden Maleinimid- bzw. N-Hydroxysuccinimidderivaten von 5-Fluorouracil in einem Lösungsmittel, vorzugsweise Dichlormethan oder THF, unter Zusatz von DMAP und einem Kondensationsmittel, i.d.R. DCC oder CMC, wobei die Kopplung über die ¹N- oder ³N-Position von 5-Fluorouracil als Carbamoyloxymethylbindung erfolgt.

Bei 5'-Desoxy-5-fluorouridin erfolgt die Synthese im einzelnen durch Umsetzen mit den unter Schritt 1 aufgeführten Säurechloriden der Maleinimid- bzw. N-Hydroxysuccinimidverbindungen der Formel V bis X zu den entsprechenden Maleinimid- bzw. N-Hydroxysuccinimidderivaten von 5'-Desoxy-5-fluorouridin in einem Lösungsmittel, vorzugsweise THF, unter eventuellem Zusatz einer tertiären Base, i.d.R. Et₃N, wobei die Kopplung über die 2'-HO- oder 3'-HO-Gruppe von 5'-Desoxy-5-fluorouridin als Esterbindung erfolgt,
oder durch Umsetzen mit den unter Schritt 1 aufgeführten Aldehyden oder Ketonen der Maleinimid- bzw. N-Hydroxysuccinimidverbindungen in einem Lösungsmittel, vorzugsweise THF, Methanol oder Ethanol, unter Zusatz einer Säure, i.d.R. Trifluoressigsäure oder p-Toluolsulfonsäure, wobei die Kopplung über die 2'-HO- und/oder 3'-HO-Gruppe von 5'-Desoxy-5-fluorouridin als Acetal- bzw. Ketalbindung erfolgt.

Bei Thioguanin erfolgt die Synthese im einzelnen durch Umsetzen mit den unter Schritt 1 aufgeführten Säurechloriden der Maleinimid- bzw. N-Hydroxysuccinimidverbindungen der Formel V bis X zu den entsprechenden Maleinimid- bzw. N-Hydroxysuccinimidderivaten von Thioguanin in einem Lösungsmittel, vorzugsweise DMF, unter eventuellern Zusatz einer tertiären Base, i.d.R. Et₃N, wobei die Kopplung über die H₂N-Gruppe von Thioguanin als Amidbindung erfolgt,
oder durch Umsetzen mit den unter Schritt 1 aufgeführten Aldehyden oder Ketonen der Maleinimid- bzw. N-Hydroxysuccinimidverbindungen in einem Lösungsmittel, vorzugsweise DMF, Methanol oder Ethanol, unter Zusatz einer Säure, i.d.R. Trifluoressigsäure oder p-Toluolsulfonsäure, wobei die Kopplung über die H₂N-Gruppe von Thioguanin als Iminbindung erfolgt.

Bei Methotrexat erfolgt die Synthese im einzelnen durch Umsetzen von Methotrexat mit den unter Schritt 1 aufgeführten Hydroxy- bzw. Aminoverbindungen von Maleinimid- bzw. N-Hydroxysuccinimidverbindungen in einem Lösungsmittel, vorzugsweise DMF oder Dimethylsulfoxid unter Zusatz von DMAP und einem Kondensationsmittel, i.d.R. DCC oder CMC, zu den entsprechenden Methotrexat-Maleinimidderivaten bzw. Methotrexat-Hydroxysuccinimidverbindungen, wobei die Kopplung entweder über die α-COOH- oder γ-COOH-Gruppe oder über beide COOH-Gruppen von Methotrexat als Ester- oder Amidbindung erfolgt,
oder durch Umsetzen von Methotrexat mit Alkylcarbazaten, i.d.R. t-Butylcarbazat, in einem Lösungsmittel, vorzugsweise DMF oder Dimethylsulfoxid unter Zusatz von DMAP und einem Kondensationsmittel, i.d.R. DCC oder CMC, und anschließender Spaltung mit Säuren, i.d.R. Trifluoressigsäure oder HCl in Ether, THF oder Dioxan, zu den entsprechenden Säurehydraziden von Methotrexat, wobei die Säurehydrazidgruppe entweder an der α-COOHoder γ-COOH-Gruppe oder an beiden COOH-Gruppen von Methotrexat eingeführt wurde, und die so erhaltenen Säurehydrazidderivate von Methotrexat nun mit einem der in Schritt 1 aufgeführten Aldehyden oder Ketonen der N-Hydroxysuccinimidverbindungen in einem Lösungsmittel, vorzugsweise DMF, Methanol, THF oder Ethanol, unter Zusatz einer Säure, i.d.R. Trifluoressigsäure oder p-Toluolsulfonsäure, zu den entsprechenden N-Hydroxysuccinimidcarboxylhydrazon-Derivaten von Methotrexat umgesetzt werden.

Bei den Taxoiden Paclitaxel und Docetaxel erfolgt die Synthese im einzelnen durch Umsetzen mit den unter Schritt 1 aufgeführten Säuren oder Säurechloriden der Maleinimid- bzw. N-Hydroxysuccinimidverbindungen der Formel V bis X zu den entsprechenden Taxoid-Maleinimidderivaten bzw. Taxoid-Hydroxysuccinimidderivaten in einem Lösungsmittel, vorzugsweise DMF oder THF unter eventuellem Zusatz einer tertiären Base, i.d.R. Et₃N, oder unter eventuellem Zusatz von DMAP und einem Kondensationsmittel, i.d.R. DCC oder CMC,
wobei die Kopplung über die C₇- oder C₁₀-OH-Gruppe des Taxoids als Esterbindung erfolgt, oder durch Umsetzen mit den mit den unter Schritt 1 aufgeführten Aminen bzw. Hydrazinen der Maleinimid- bzw. N-Hydroxysuccinimidverbindungen in einem Lösungsmittel, vorzugsweise DMF, Methanol oder Ethanol, unter Zusatz einer Säure, i.d.R. p-Toluolsulfonsäure bzw. Trifluoressigsäure, wobei die Kopplung über die C₉-Ketoposition des Taxoids als Imin- bzw. Hydrazonbindung erfolgt,
oder durch Umsetzen mit den unter Schritt 1 aufgeführten Säurehydraziden der Maleinimid- bzw. N-Hydroxysuccinimidverbindungen in einem Lösungsmittel, vorzugsweise DMF, Methanol oder Ethanol, unter Zusatz einer Säure, i.d.R. p-Toluolsulfonsäure bzw. Trifluoressigsäure, wobei die Kopplung über die C₉-Ketoposition des Taxoids als Carboxyloder Sulfonylhydrazonbindung erfolgt.

Bei den Camptothecinen Topotecan bzw. 9-Aminocamptothecin erfolgt die Synthese im einzelnen durch Umsetzen mit den unter Schritt 1 aufgeführten Säuren oder Säurechloriden der Maleinimid- bzw. N-Hydroxysuccinimidverbindungen der Formel V bis X zu den entsprechenden Taxoid-Maleinimidderivaten bzw. Taxoid-Hydroxysuccinimidderivaten in einem Lösungsmittel, vorzugsweise DMF oder THF unter eventuellem Zusatz einer tertiären Base, i.d.R. Et₃N, oder unter eventuellem Zusatz von DMAP und einem Kondensationsmittel, i.d.R DCC oder CMC, wobei die Kopplung über die C₁₀-OH-Gruppe des Topetecans als Esterbindung bzw. über die C₉-NH₂-Gruppe des 9-Aminocamptothecins als Amidbindung erfolgt,
oder durch Umsetzen von 9-Aminocamptothecin mit den unter Schritt 1 aufgeführten Aldehyden bzw. Ketonen der Maleinimid- bzw. N-Hydroxysuccinimidverbindungen in einem Lösungsmittel, vorzugsweise DMF, Methanol oder Ethanol, unter eventuellem Zusatz einer Säure, i.d.R. p-Toluolsulfonsäure bzw. Trifluoressigsäure, wobei die Kopplung über die C₉-NH₂-Gruppe als Iminbindung erfolgt.

Bei den Podophyllotoxinderivaten Etoposid, Teniposid und Mitopodozid erfolgt die Synthese im einzelnen durch Umsetzen mit den unter Schritt 1 aufgeführten Säuren oder Säurechloriden der Maleinimid- bzw. N-Hydroxysuccinimidverbindungen der Formel V bis X zu den entsprechenden Taxoid-Maleinimidderivaten bzw. Taxoid-Hydroxysuccinimidderivaten in einem Lösungsmittel, vorzugsweise DMF, Dichlormethan oder THF unter eventuellem Zusatz einer tertiären Base, i.d.R. Et₃N, oder unter eventuellem Zusatz von DMAP und einem Kondensationsmittel, i.d.R. DCC oder CMC, wobei die Kopplung über eine der beiden aliphatischen HO-Gruppen des Podophyllotoxinderivats als Esterbindung erfolgt.

Bei den Vinca-Alkaloiden Vinblastin, Vincristin, Vindesin und Vinorelbin erfolgt die Synthese im einzelnen durch Umsetzen mit den unter Schritt 1 aufgeführten Säuren oder Säurechloriden der Maleinimid- bzw. N-Hydroxysuccinimidverbindungen der Formel V bis X zu den entsprechenden Taxoid-Maleinimidderivaten bzw. Taxoid-Hydroxysuccinimidderivaten in einem Lösungsmittel, vorzugsweise DMF, Dichlormethan oder THF unter eventuellem Zusatz einer tertiären Base, i.d.R. Et₃N, oder unter eventuellem Zusatz von DMAP und einem Kondensationsmittel, i.d.R. DCC oder CMC, wobei die Kopplung über eine der aliphatischen HO-Gruppen des Vinca-Alkaloids als Esterbindung erfolgt.

Bei den Maleinimid- bzw. N-Hydroxysuccinimidderivaten der cis-konfigurierten Platin(II)-Komplexe erfolgt die Synthese im einzelnen durch Umsetzen der entsprechenden Aminoverbindungen H₂N-CH₂CH₂-NH-(CH₂)ₙ-X, (H₂N-CH₂)₂CH-(CH₂)ₙ-X oder H₂N-CH₂CH(NH₂)-(CH₂)ₙ-X (allgemeine Formel I, II und III), wobei eine oder zwei der primären bzw. sekundären Aminogruppen mit einer BOC-Gruppe geschützt wurde (Umsetzung mit Bistert.-butyloxycarbonylanhydrid) und X -NH₂ bzw. -OH ist, mit den unter Schritt 1 aufgeführten Säuren oder Säurechloriden der Maleinimid- bzw. N-Hydroxysuccinimidverbindungen der allgemeinen Formeln V-X in einem Lösungsmittel, vorzugsweise THF oder Ethylacetat, unter eventuellem Zusatz einer tertiären Base, i.d.R. Et₃N, oder unter eventuellem Zusatz von DMAP und einem Kondensationsmittel, i.d.R. DCC oder CMC, zu den entsprechenden BOC-geschützten Maleinimid- bzw. N-Hydroxysuccinimidderivaten, die nun durch Trifluoressigsäure oder HCl in Ether, THF oder Dioxan unter Abspaltung der BOC-Gruppe in die entsprechenden Trifluoracetate bzw. Hydrochloride und schließlich durch Umsetzen mit einem Tetrachloroplatinat(II)-Salz, vorzugsweise Kaliumtetrachloroplatinat(II), in Wasser, Salzpuffern, DMF, DMF/Wasser-Gemischen, THF/Wasser-Gemischen oder DMF/Methanol-Gemischen, in die entsprechenden Platin(II)-Komplexe übergeführt werden, wobei die Kopplung über die endständige HO-Gruppe als Esterbindung oder über die endständige H₂N-Gruppe als Säureamidbindung erfolgt,
oder durch Umsetzen der entsprechenden Aminoverbindungen H₂N-CH₂CH₂-NH-(CH₂)ₙ-X, (H₂N-CH₂)₂CH-(CH₂)ₙ-X oder H₂N-CH₂CH(NH₂)-(CH₂)ₙ-X (allgemeine Formel I, II und III), wobei eine oder zwei der primären bzw. sekundären Aminogruppen mit einer BOC-Gruppe geschützt wurde (Umsetzung mit Bis-tert.-butyloxycarbonylanhydrid) und X -NH₂ bzw. -OH ist, mit Verbindungen des Typs HOOC-R-COCR* oder ClOC-R-COCR* (R ist eine aliphatische Kohlenstoffkette mit 1-6 Kohlenstoffatomen oder eine substituierte bzw. unsubstituierte Phenylengruppe, und R* ist H, Phenyl, Alkyl mit 1-6 Kohlenstoffatomen) in einem Lösungsmittel, vorzugsweise THF oder Ethylacetat, unter eventuellem Zusatz einer tertiären Base, i.d.R. Et₃N, oder unter eventuellem Zusatz von DMAP und einem Kondensationsmittel, i.d.R. DCC oder CMC, zu den entsprechenden BOC-geschützten Maleinimid- bzw. N-Hydroxysuccinimidderivaten, die nun eine weitere Carbonylfunktion enthalten, die im folgenden mit einem der in Schritt 1 aufgeführten Aminen, Säurehydraziden oder Hydrazinen der Maleinimid- bzw. N-Hydroxysuccinimidverbindungen in einem Lösungsmittel, vorzugsweise DMF, Methanol oder Ethanol, unter Zusatz einer Säure, i.d.R. p-Toluolsulfonsäure oder Trifluoressigsäure, zu den entsprechenden Imin-, Carboxylhydrazonoder Hydrazon-Derivaten umgesetzt werden, die nun wiederum durch Trifluoressigsäure oder HCl in Ether, THF oder Dioxan unter Abspaltung der BOC-Gruppe in die entsprechenden Trifluoracetate bzw. Hydrochloride und schließlich durch Umsetzen mit einem Tetrachloroplatinat(II)-Salz, vorzugsweise Kaliumtetrachloroplatinat(II), in Wasser, Salzpuffern, DMF, DMF/Wasser-Gemischen, THF/Wasser-Gemischen oder DMF/Methanol-Gemischen, in die entsprechenden Platin(II)-Komplexe übergeführt werden,
oder durch Umsetzen der entsprechenden Aminoverbindungen H₂N-CH₂CH₂-NH-(CH₂)ₙ-X, (H₂N-CH₂)₂CH-(CH₂)ₙ-X oder H₂N-CH₂CH(NH₂)-(CH₂)ₙ-X (allgemeine Formel I, II und III), wobei eine oder zwei der primären bzw. sekundären Aminogruppen mit einer BOC-Gruppe geschützt wurde (Umsetzung mit Bis-tert.-butyloxycarbonylanhydrid) und X -COOH ist bzw. diese Carboxylgruppe durch Säurehalogenierungsreagenzien, wie Thionylchlorid oder Oxalychlorid, in das Säurechlorid übergeführt wurde, mit Verbindungen des Typs HOR-COCR* oder H₂N-R-COCR^{*} (R ist eine aliphatische Kohlenstoffkette mit 1-6 Kohlenstoffatomen oder eine substituierte bzw. unsubstituierte Phenylengruppe, und R* ist H, Phenyl, Alkyl mit 1-6 Kohlenstoffatomen) in einem Lösungsmittel, vorzugsweise THF oder Ethylacetat, unter eventuellem Zusatz einer tertiären Base, i.d.R. Et₃N, oder unter eventuellem Zusatz von DMAP und einem Kondensationsmittel, i.d.R. DCC oder CMC, zu den entsprechenden BOC-geschützten Maleinimid- bzw. N-Hydroxysuccinimidderivaten, die nun eine weitere Carbonylfunktion enthalten, die im folgenden mit einem der in Schritt 1 aufgeführten Aminen, Säurehydraziden oder Hydrazinen der Maleinimid- bzw. N-Hydroxysuccinimidverbindungen in einem Lösungsmittel, vorzugsweise DMF, Methanol oder Ethanol, unter Zusatz einer Säure, i.d.R. p-Toluolsulfonsäure oder Trifluoressigsäure, zu den entsprechenden Imin-, Carboxylhydrazon- oder Hydrazon-Derivaten umgesetzt werden, die nun wiederum durch Trifluoressigsäure oder HCl in Ether, THF oder Dioxan unter Abspaltung der BOC-Gruppe in die entsprechenden Trifluoracetate bzw. Hydrochloride und schließlich durch Umsetzen mit einem Tetrachloroplatinat(II)-Salz, vorzugsweise Kaliumtetrachloroplatinat(II), in Wasser, Salzpuffern, DMF, DMF/Wasser-Gemischen, THF/Wasser-Gemischen oder DMF/Methanol-Gemischen, in die entsprechenden Platin(II)-Komplexe übergeführt werden.

Bei den Maleinimid- bzw. N-Hydroxysuccinimidderivaten mit den Malonsäurederivaten der allgemeinen Formel IV (HOOC)₂CH-(CH₂)n-X zu den *cis*-konfigurierten Ptatin(II)-Kompiexen erfolgt die Synthese analog zu den oben beschriebenen Komplexen, wobei die Platin(II)-Komplexe dadurch erhalten werden, daß die Maleinimid- bzw. N-Hydroxysuccinimidderivate der Malonsäurederivate mit *cis*-[PtA₂B] (A = Halogen, vorzugweise Cl oder J, B = (NH₃)₂, Ethylendiamin, Propandiamin, 1,2-Diaminocyclohexan) zu den entsprechenden Platin(II)-Komplexen in einem Lösungsmittel, wie Wasser, Salzpuffern, DMF, DMF/Wasser-Gemischen, THF/Wasser-Gemischen oder DMF/Methanol-Gemischen unter Zugabe einer Hydroxidlösung, vorzugweise wässrige KOH, umgesetzt werden. Die Reaktion kann gegebenenfalls in Gegenwart von Silbernitat (AgNO₃) oder Silbersulfat (Ag₂SO₄) durchgeführt werden. Der Platinkomplex wird durch Kristallisation oder durch Zugabe von einem Lösungsmittel, vorzugweise Diethylether oder THF, erhalten.

Die Isolierung der oben genannten Maleinimid- bzw. N-Hydroxysuccinimidderivate zytostatischer Verbindungen erfolgt entweder durch Kristallisation, durch Kieselgelsäulenchromatographie oder durch präparative HPLC bzw. LPLC auf einer reversephase(C8 oder C18)- oder Diol-Säule, wie in den untenstehenden Beispielen beschrieben.

### Schritt 3 : Thiolierung des Trägerprotein

Sulfhydrylgruppen (HS-Gruppen) werden durch Umsetzen des Trägerproteins mit einem Thiolierungsreagenz, vorzugsweise Iminothiolan, in humanes Serumtransferrin und Serumalbumin, eingeführt. Die Thiolierung erfolgt in einem Salzpuffer, i.d.R. in 0,1 M Natriumborat, 0,15 M NaCl, 0,001 M EDTA - pH = 8,0, mit einem Überschuß an Thiolierungsreagenz (2- bis 100-facher Überschuß) und anschließender Gelfiltration (beispielsweise Sephadex® G10 oder G25) mit einem Salzpuffer, wie 0,025 M Natriumborat, 0,15 M NaCl - pH 6,0 - 7,5 oder 0,004 M Phosphat, 0,15 M NaCl - pH 6,0 - 7,5. Die Konzentration an Protein nach erfolgter Gelfiltration wird durch die Extinktionskoeffizienten bei 280.nm bestimmt und liegt i.d.R. zwischen 1,0 x 10⁻⁴ und 5,0 x 10⁻³ M. Die Anzahl der eingeführten HS-Gruppen wird mit Ellmann's Reagenz bei 412 nm bestimmt. Durch Variation der Reaktionsbedingungen können im Mittel 1 bis 30 HS-Gruppen eingeführt werden. Die thiolierte Transferrin- bzw. Albuminlösung wird direkt für die Synthese der Konjugate eingesetzt.

### Schritt 4: Kopplung der zytostatischen Maleinimid- bzw. N-Hydroxysuccinimidverbindungen an das native oder thiolierte Trägerprotein

*Kopplung der zytostatischen Maleinimidderivate an das thiolierte Trägerprotein*. Die zytostatischen Maleinimidderivate (s. Schritt 2) werden mit thioliertem Albumin (s. Schritt 3) bei Raumtemperatur umgesetzt. Dabei wird zu dem thiolierten Protein, das sich in einem entgasten Salzpuffer, wie 0,025 M Natriumborat, 0.15 M NaCl - pH 6,0 - 7,5 oder 0,004 M Phosphat, 0,15 M NaCl - pH 6,0 - 7,5, befindet, ein etwa 1,1- bis 10-facher Überschuß des zytostatischen Maleinimidderivats (bezogen auf die Anzahl der vorhandenen HS-Gruppen im Protein, gelöst in einer minimalen Menge Lösemittel, i.d.R. DMF, Dimethylsulfoxid, Wasser, Ethanol, Methanol, Acetonitril oder THF (etwa 1 - 10% des Volumens der thiolierten Probe), gegeben. Nach etwa 5 bis 120 Minuten wird die Lösung zentrifugiert, und das gebildete Proteinkonjugat wird durch anschließende Gelfiltration (beispielsweise Sephadex® G10, G25 oder LH20) in einem entgasten Salzpuffer, wie 0,025 M Natriumborat, 0,15 M NaCl - pH 6,0 - 7,5, 0,004 M Phosphat, 0,15 M NaCl - pH 6,0 - 7,5, oder 0,1-0,2 M NaHCO₃, oder in Methanol oder THF vom überschüssigen zytostatischen Maleinimidderivat abgetrennt. Es kann vorteilhaft sein, die thiolierte Proteinlösung vor Zugabe des Maleinimidderivats mit einem Salzpuffer zu verdünnen und das Maleinimidderivat, gelöst in einer minimalen Menge Lösemittel, zuzugeben und anschließend nach etwa 5-20 Minuten die Lösung mit einem handelsüblichen Konzentrator aufzukonzentrieren und das Proteinkonjugat, wie oben beschrieben, zu isolieren. Des weiteren kann die Lösung des so erhaltenen Proteinkonjugats mit einem handelsüblichen Konzentrator aufkonzentriert oder das Lösungsmittel im Hochvakuum entfernt werden.

*Kopplung der zytostatischen N-Hydroxysuccinimidderivate an das native Trägerprotein:* Die zytostatischen N-Hydoxysuccinimidderivate (s. Schritt 2) werden mit Albumin bei Raumtemperatur umgesetzt. Dabei wird zu dem Protein, das sich in einem entgasten Salzpuffer, wie 0,025 M Natriumborat, 0.15 M NaCl - pH 6,0 - 8,0 oder 0,004 M Phosphat, 0. 15 M NaCl - pH 6,0 - 8,0, befindet, ein etwa 1,1- bis 50-facher Überschuß des zytostatischen N-Hydoxysuccinimidderivats, gelöst in einer minimalen Menge Lösemittel, i.d.R. DMF, Dimethylsulfoxid, Wasser, Ethanol, Methanol, Acetonitril oder THF (etwa 1 - 10% des Volumens der thiolierten Probe), gegeben. Nach etwa 5 Minuten bis 48 Stunden wird die Lösung zentrifugiert, und das gebildete Proteinkonjugat wird durch anschließende Gelfiltration (beispielsweise Sephadex® G10,G25 oder LH20) in einem entgasten Salzpuffer, wie 0,025 M Natriumborat, 0.15 M NaCl - pH 6,0 - 7.5, 0,004 M Phosphat, 0,15 M NaCl - pH 6,0 - 7,5 oder 0,1-0,2 M NaHCO₃, oder in Methanol oder THF vom überschüssigen zytostatischen N-Hydoxysuccinimidderivat abgetrennt. Die Lösung des so erhaltenen Proteinkonjugats kann mit einem handelsüblichen Konzentrator aufkonzentriert oder das Lösungsmittel im Hochvakuum entfernt werden.

Die Anzahl der an das Trägerprotein gebundenen zytostatischen Maleinimidderivate bzw. N-Hydroxysuccinimidderivate erfolgt entweder durch eine photometrische Konzentrationsbestimmung bei der absorbierenden Wellenlänge des zytostatischen Maleinimid- bzw. N-Hydroxysuccinimidderivats (typischerweise zwischen 220 und 600 nm) und/oder durch eine colorimetrische Bestimmung, die bei den Konjugaten von Chlorambucil und Melphalan mit Hilfe des NBP-Tests (Epstein, J. Rosenthal, R.W., Ess, R.J. *Anal. Chem.* (1955), 27, 1435-1439), bei den Konjugaten von 5-Fluorouracil und 5'-Desoxy-5-fluorouridin mit Hilfe eines Assays nach Habib (Habib, S.T., *Talanta* (1981), 28, 685-87) und bei den Konjugaten der *cis*-konfigurierten Platin(II)-Analoga mit Hilfe einer Bestimmung nach Gonias und Pizzo (Gonias, S.L., Pizzo, S.V. *J.Biol.Chem.* (1982), 258, 5764-5769) oder der Atomabsorptionsspektroskopie (AAS) erfolgt.

Im Mittel werden auf dem oben beschrieben Weg etwa 1-30 Moleküle der zytostatischen Verbindung an ein Molekül Protein gebunden. Die Reinheit der Proteinkonjugate bzw. PEG-Konjugate wird durch HPLC mit Hilfe einer analytischen Säule (Bio-Sil SEC 250, (300 mm x 7.8 mm) von Bio-RAD, mobile Phase: i.d.R. 0.15 M NaCl, 0.01 M NaH₂PO₄, 5% CH₃CN - pH 7.0 oder Nucleogel® aqua-OH 40 bzw. 60, von Machery und Nagel, mobile Phase: i.d.R. 0.1 M NaCl, 0.004 M NaH₂PO₄, 30% Methanol- pH 7.0) geprüft. Dabei besitzen die Albumin konjugate eine Reinheit von > 90%.

Die Proteinkonjugate bzw. PEG-Konjugate können in gelöster Form bei 0-5°C, in gefrorener Form bei T = -20°C bzw. -78°C gelagert werden. Des weiteren ist es möglich, die Lösung der Konjugate zu lyophilisieren und das Lyophilisat bei +5 bis -78°C zu lagern.

Gegenstand der Erfindung sind solche Chemoimmunokonjugate bestehend aus Albumin, das gemäß Anspruch 1 mit etwa zwei bis dreißig Äquivalenten einer zytostatischen Verbindung beladen ist und mit einem Protein, beispielsweise mit Transferrin oder mit einem monoklonalen Antikörper, der gegen ein tumorassoziiertes Antigen gerichtet ist, vorzugsweise aber mit Transferrin, über eine der unter Anspruch 5 genannten Bismaleinimidverbindungen oder über eine aliphatische bzw. aromatische Bismaleinimidverbindung konjugiert ist. Dabei werden etwa 80-90 % der in Albumin eingeführten Thiolgruppen mit dem zytostatischen Maleinimidderivat, gelöst in einer minimalem Menge Lösemittel, i.d.R. DMF, Dimethylsulfoxid, Ethanol, Methanol, Acetonitril oder THF (etwa 1 - 10% des Volumens der thiolierten Probe), umgesetzt und nach etwa 5 bis 60 Minuten ein 1,5- bis 20-facher Überschuß der Bismaleinimidverbindung, gelöst in einer minimalem Menge Lösemittel, i.d.R. DMF, Dimethylsulfoxid, Ethanol, Methanol, Acetonitril oder THF (etwa 1 - 10% des Volumens der thiolierten Probe), zugegeben. Nach etwa 5 bis 20 Minuten wird die Lösung zentrifugiert, und das gebildete Proteinkonjugat wird durch anschließende Gelfiltration (beispielsweise Sephadex®) G10 oder G25) in einem Salzpuffer, i.d.R. 0.025 M Natriumborat, 0.15 M NaCl - pH 6,0-7.5 oder 0,004 M Phosphat, 0,15 M NaCl - pH 6,0 - 7,5, von dem zytostatischen Maleinimidderivat und der Bismaleinimidverbindung abgetrennt. Anschließend wird das so modifizierte Albuminlkonjugat mit einem der oben genannten Proteine, welches im Mittel etwa 1,5 Thiolgruppen enthält, umgesetzt und das resultierende Chemoimmunokonjugat, das nun aus einem mit zytostatischen Verbindungen beladenen Albuminmolekül und dem oben genannten Protein besteht, mit Hilfe einer Superdex-200-Säule (Fa. Pharmacia) oder einer Hydroxylapatitsäule (Fa. Pharmacia oder Bio-Rad) in einem Salzpuffer, i.d.R. 0,025 M Natriumborat, 0.15 M NaCl - pH 6,5-8.0, 0,004 M Phosphat, 0,15 M NaCl - pH 6,5 - 8,0, isoliert.

Es ist auch möglich, zunächst eines der oben genannten Proteine, welches im Mittel 1,5 Thiolgruppen enthält, mit einem 1,5- bis 10-fachen Überschuß der Bismaleinimidverbindung umzusetzen und das gebildete Proteinkonjugat durch anschließende Gelfiltration (beispielsweise Sephadex G10 oder G25) mit einem entgasten Salzpuffer, wie 0,025 M Natriumborat, 0,15 M NaCl - pH 6,0 - 7,5, 0,004 M Phosphat, 0,15 M NaCl - pH 6,0 - 7,5, oder 0,1-0,2 M NaHCO₃, von der Bismaleinimidverbindung abzutrennen, und anschließend das so modifizierte Proteinkonjugat, das nun im Mittel 1,5 Äquivalente der Bismaleinimidverbindung enthält, mit modifiziertem Albuminkonjugat umzusetzen, wobei etwa 80-90% der in Albumin eingeführten Thiolgruppen mit einem zytostatischen Maleinimidderivat bereits umgesetzt wurden. Das resultierende Chemoimmunokonjugat, das nun aus einem mit zytostatischen Verbindungen beladenen Albuminmolekül und dem oben genannten Protein besteht, wird mit Hilfe einer Superdex-200-Säule (Fa. Pharmacia) oder einer Hydroxylapatitsäule (Fa. Pharmacia oder Bio-Rad) in einem entgasten Salzpuffer, wie 0,025 M Natriumborat, 0,15 M NaCl - pH 6,0 - 8,0, 0,004 M Phosphat, 0,15 M NaCl - pH 6,0 - 8,0, oder 0,1-0,2 M NaHCO₃, isoliert.

Die folgenden Beispiele erläutern die Erfindung näher.

### Ausfübrungsbeispiele

### Schritt 1: Synthese von Maleinimid- und Hydroxysuccinimidverbindungen

### p-Maleinimidobenzophenon

14,0 g (70 mmol) p-Aminobenzophenon wurden in 70 ml Aceton gelöst und 7,0 g (70 mmol) Maleinsäureanhydrid, gelöst in 40 ml Aceton, über einen Zeitraum von 15 Minuten bei Raumtemperatur zugetropft. Der Ansatz wurde 3 h lang bei Raumtemperatur gerührt. Anschließend wurde der Niederschlag abfiltriert, mit Ether gewaschen und im Vakuum getrocknet (Ausbeute: 93%). 19,3 g (65,4 mmol) N-(p-Benzophenyl)-maleinsäureamid und 2,6 g (31,0 mmol) wasserfreies Natriumacetat wurden bei 55°C in 50 ml Essigsäureanhydrid gelöst und bei dieser Temperatur 2 h lang gerührt. Anschließend wurde das Essigsäureanhydrid bei 60°C im Wasserstrahlvakuum entfernt. Zum Rückstand wurden 300 ml Wasser gegeben und 2h lang bei 70°C gerührt. Der während dieser Zeit ausgefallene Niederschlag wurde abgesaugt, mit Wasser gewaschen und aus Aceton umkristallisiert (Ausbeute: 94%); Schmelzpunkt: 150°C; R_{f}-Wert: 0,70 (Essigester/Hexan 6/1); berechnet: C: 73,64 % H: 3,97 % N: 5,05 %, C₁₇H₁₁NO₃; gefunden: C: 73,15 % H: 3,96 % N: 5,00%

### p-Maleinimidophenylessigsäure

25,0 g (166,9 mmol) p-Aminophenylessigsäure wurden in 250 ml Methanol suspendiert und in der Wärme unter Zugabe von 500 ml Aceton in Lösung gebracht. 19,25 g (166,9 mmol) Maleinsäureanhydrid wurden in 100 ml Aceton gelöst und während 60 Minuten in die vorher auf unter 40°C abgekühlte Lösung zugetropft. Der Ansatz wurde 60 Minuten lang bei Raumtemperatur gerührt. Anschließend wurde die Reaktionsmischung auf etwa 300 ml eingeengt und bei -20°C kaltgestellt. Der Niederschlag wurde abgesaugt, mit Aceton gewaschen und im Hochvakuum getrocknet (Ausbeute: 82%). 18,0 g (72 mmol) N-(4-Essigsäurephenyl)maleinsäureamid wurden in 2,4 l Toluol suspendiert und zum Rückfluß erhitzt. In der Wärme wurden 14,7 g (20,2 ml, 144 mmol) Triethylamin zugegeben und anschließend 2 h lang unter Rückfluß am Wasserabscheider erhitzt. Dann wurde vom entstandenen roten Öl abdekantiert und das Lösungsmittel im Vakuum entfernt. Der gelbe Rückstand wurde in 300 ml Wasser aufgenommen und mit 1 M HCl auf pH = 2 eingestellt. Die saure Lösung wurde mit Essigester extrahiert (6 x 50 ml). Die vereinigten Essigester-Phasen wurden über Natriumsulfat getrocknet und das Lösungsmittel im Vakuum entfernt. Das Produkt wurde aus Essigester/Hexan umkristallisiert (Ausbeute: 51% gelbe Kristalle); Schmelzpunkt: 158°C; R_{f}-Wert: 0,45 (Essigester/Methanol 2/1); berechnet: C: 62,34 % H: 3,92% N: 6,09% (C₁₂H₉NO₄); gefunden: C: 61,84% H: 4,43 % N: 5,59 %

### p-Maleinimidophenylessigsäurechlorid

1,0 g (4,33 mmol) p-Maleinimidophenylessigsäure wurden in 25 ml Dichlormethan suspendiert und mit einem 2,5-fachen Überschuß an Oxalsäuredichlorid (1,37 g, 945 µl; 10,82 mmol) versetzt. Die Reaktionsmischung wurde unter Feuchtigskeitsausschluß auf 30-40°C erwärmt und 15 h lang gerührt. Anschließend wurde das Lösungsmittel im Vakuum entfernt und im Hochvakuum getrocknet. Kristallisation aus Toluol lieferte ein gelbes Pulver (Ausbeute: 59%); Schmelzpunkt: 154°C; R_{f}-Wert: 0,29 (Essigester/Hexan 4/1)

| | | | | | |
|---|---|---|---|---|---|
| Elementaranalyse: (C₁₂H₈NO₃Cl) | berechnet | C: 58,91% | H: 3,30 % | N: 5,75% | Cl: 14,49 % |
| | gefunden | C: 60,61% | H: 3,64% | N: 5,13% | Cl: 13,80% |

### p-Maleinimidophenylessigsäurehydrazid·CF₃COOH

3,5 g (14 mmol) p-Maleinimidophenylessigsäurechlorid wurden zusammen mit tert.-Butylcarbazat (2,87 g, 21,7 mmol) in 50 ml wasserfreiem Tetrahydrofuran gelöst und 2 h lang bei Raumtemperatur gerührt. Das Tetrahydrofuran wurde im Hochvakuum entfernt und der Rückstand in 500 ml Essigester aufgenommen. Die Essigesterphase wurde zweimal mit je 125 ml Wasser ausgeschüttelt und anschließend über wasserfreiem Natriumsulfat getrocknet. Die Lösung wurde im Vakuum eingedampft und aus Essigester/Hexan umkristallisiert. Das Produkt wurde abgesaugt und im Hochvakuum getrocknet (Ausbeute: 90 %); Schmelzpunkt: Zersetzung ab 152°C; R_{f} -Wert: 0,50 (Tetrahydrofuran/Hexan 3/1). 2,5 g (7,25 mmol) p-Maleinimidophenylessigsäurehydrazino-tert.-butylcarbazat wurden in 12 ml Trifluoressigsäure gelöst und 1h lang bei Raumtemperatur gerührt. Die Trifluoressigsäure wurde anschließend im Hochvakuum entfernt und der Rückstand in 50 ml Ether suspendiert. Der Niederschlag wurde abgesaugt, mit trockenem Ether gewaschen und im Hochvakuum getrocknet (Ausbeute: 82 % gelbliches Pulver); Schmelzpunkt: 112°C; R_{f}-Wert: 0,06 (Tetrahydrofuran/Hexan 3/1)

| | | | | |
|---|---|---|---|---|
| (C₁₄H₁₂N₃O₅F₃) | berechnet | C: 46,80 % | H: 3,34 % | N: 11,70 % |
| | gefunden | C: 46,95 % | H: 3,24 % | N: 11,51 % |

### Maleinimidoacetaldehyd

91,6 g (100 ml, 689 mmol) Aminoacetaldehyddiethylacetal wurden in 200 ml Essigester gelöst. Danach wurden 67,5 g (689 mmol) Maleinsäureanhydrid, gelöst in 200 ml Essigester, unter Rühren und Eiskühlung innerhalb 60 Minuten zugetropft. Es wurde 1 h lang bei Raumtemperatur gerührt. Dann wurde das Reaktionsgemisch auf die Hälfte eingeengt und bei-20°C kaltgestellt. Nach 24 h wurde der entstandene Niederschlag im Vakuum abgesaugt, mit Essigester und Ether gewaschen, und anschließend im Hochvakuum getrocknet. (Ausbeute: 90%). 30,95 g (133,8 mmol) N-(Acetaldehyddiethylacetal)maleinsäureamid wurden in 900 ml Toluol gelöst, 14,2 g (19,6 ml, 140,5 mmol) Triethylamin hinzugefügt und das Reaktionsgemisch 15 h lang am Wasserabscheider gekocht. Danach wurde das Lösungsmittel im Vakuum entfernt. Der zurückbleibende Sirup wurde in 350 ml Diethylether aufgenommen und 3 x mit je 50 ml Wasser extrahiert. Die etherische Phase wurde über Na₂SO₄ getrocknet, das Lösungsmittel im Vakuum entfernt und der Rückstand säulenchromatographisch gereinigt (Essigester/Hexan 1/1; Ausbeute: 36 % farbloser Sirup); R_{f}-Wert: 0,49 (Essigester/Hexan 1/1)

| | | | | |
|---|---|---|---|---|
| (C₁₀H₁₅NO₄) | berechnet | C: 58,15% | H: 7,49 % | N: 6,17% |
| | gefunden | C: 58,10 % | H: 6,98 % | N: 6,35% |

20,1 g (94,3 mmol) Maleinimidoacetaldehyddiethylacetal wurden in 350 ml Dichlormethan gelöst und 40,2 g Kieselgel 60 unter Rühren hinzugefügt. Es wurden 4,0 g 30%ige Schwefelsäure zugegeben und 60 h lang unter Rückfluß erhitzt. Dann wurde das Kieselgel abfiltriert und die Reaktionslösung mit 4 x 50 ml Wasser extrahiert. Das Dichlormethan wurde über Natriumsulfat getrocknet und eingedampft. Der zurückbleibende Sirup wurde aus Essigester/Hexan (1:10) umkristallisiert (Ausbeute: 15% weiße Kristalle); Schmelzpunkt: 68-

| | | | | |
|---|---|---|---|---|
| 69°C; R_{f}-Wert: 0,52 (THF/Hexan 3/1) (C₆H₅NO₃) | berechnet | C: 51,76 % | H: 3,62 % | N: 10,06 % |
| | gefunden | C: 51,48 % | H: 4,14 % | N: 9,60 % |

### Chlorameisensäure-2-maleinimidoethylester

2,0 g (14,2 mmol) 2-Hydroxyethylmaleinimid wurden in 150 ml absoluten Dichlormethan gelöst und mit 1,56 g (4,8 mmol) Triphosgen versetzt. Nach Zugabe von 479 mg (660 µl; 4,8 mmol) Triethylamin wurde der Ansatz 72 h lang bei Raumtemperatur gerührt, anschließend das Lösungsmittel im Vakuum entfernt und der Rückstand säulenchromatographisch über Kieselgel gereinigt (Laufmittel: Essigester/Hexan 2/1; Ausbeute: 80 % farbloser Feststoff);Schmelzpunkt: 45 °C; R_{f}-Wert: 0,69 (Essigester/Hexan 2/1); berechnet: C: 41,25% H: 2,95% N: 6,88% Cl: 17,41% (C₇H₆NO₄Cl); gefunden: C: 41,11% H: 3,00 % N: 6,88% Cl: 16,94 %

### Synthese von Bismaleinimidverbindungen:

### 1,4-Diamino-N,N'-di-m-maleinimidobenzyl-butan

2,36 g (10 mmol) Maleinimidobenzoesäurechlorid sowie 0,44 g (5 mmol) 1,4-Diaminobutan wurden jeweils in 60 ml Ethylacetat gelöst. In einem Dreihalskolben wurden 40 ml Ethylacetat vorgelegt. Unter Rühren bei Raumtemperatur wurden die beiden Lösungen synchron über einen Zeitraum von 30 min zugetropft. Während des Zutropfens fiel ein hellgelber Niederschlag aus. Der Ansatz wurde 2 h lang gerührt, der Niederschlag abgesaugt und mit Diethylether gewaschen. Der hellgelbe Feststoff wurde aus Aceton umkristallisiert, mit Wasser und anschließend nochmals mit Ether gewaschen und i. Vak. getrocknet. Man erhielt 2,1 g (3,9 mmol, 75% d. Theorie) des Produktes als gelb-weißen Feststoff, DC: Kieselgel, THF/Hexan 4:1, R_{f} = 0,30, Fp.: 221°C (Zersetzung); C₂₆H₂₂N₄O₆ (486 g/mol) berechnet: C: 64,20%; H: 4,53%; N: 11,52%; O: 19.75%; gefunden: C: 62,57%; H: 4,55%; N: 10,67%

### Diacylhydrazon aus Adipinsäuredihydrazid und m-Maleinimidoacetophenon

1,0 g (4,65 mmol) m-Maleinimidoacetophenon wurden zusammen mit 368 mg (2,11 mmol) Adipinsäuredihydrazid in 40 ml absolutem Methanol gelöst. Dem Reaktionsansatz wurden 100 µl Trifluoressigsäure hinzugegeben und 15 h bei RT gerührt. Der dabei ausgefallene Niederschlag wurde abgesaugt und 2 x mit je 30 ml Methanol sowie 4 x mit je 50 ml Diethylether gewaschen. Anschließend wurde das Produkt i. Vak. getrocknet; DC: Kieselgel, THF/Hexan 4: 1, R_{f} = 0.36, Fp.: 240 °C (Zersetzung); C₃₀H₂₈N₆O₆ (568 g/mol) berechnet:C: 63,38 %, H: 4,93 %, N: 14,79 %; gefunden: C: 63,18 %, H: 4,83 %, N: 15,03 %

### Synthese von N-Hydroxysuccinimidester-Verbindungen

### 4-Acetophenoncarbonsäure-(N-hydroxysuccinimid)-ester

2,2 g (1,2 mmol) Acetophenon-4-carbonsäure und 1,52 g (1,3 mmol) und 20 mg DMAP werden in 40 ml Tetrahydrofuran gelöst und anschließend 2,7 g DCC, gelöst in 20 ml THF, in der Kälte innerhalb von 1 h zugetropft. Die Reaktionsmischung wird in der Kälte während 12 h gerührt, filtriert, THF im Vakuum entfernt und der Rückstand aus Essigester/Hexan umkristallisiert (Ausbeute 65 %). DC: Kieselgel, Essigester/Methanol 1:1, R_{f} = 0.71, Fp.: 140 °C, C₁₃H₁₁NO₅ (261 g/mol); berechnet: C: 59,77 %, H: 4,21 %, N: 5.36 %; gefunden: C: 60,1 %, H: 4,2 %, N: 5,0 %

### 4-Carboxybenzoylhvdrazid

25 g (138.9 mmol) Terephthalsäuremonomethylester werden in 200 ml THF suspendiert und zum Rückfluß erhitzt. Bei der portionsweisen Zugabe von 40.5 ml (833.4 mmol, 6 eq) Hydrazinmonohydrat fällt das Produkt weißer Feststoff aus. Das überstehende THF wird abdekantiert und der Rückstand in Wasser gelöst. Durch Zugabe von konz. HCl wird ein pH von etwa 4 eingestellt. Es bildet sich ein weißer Niederschlag, der abgesaugt und mit halbkonz. HCl gewaschen wird. Das Produkt wird i. Vak. getrocknet und aus methanolischer Natronlauge umkristallisiert. Man erhält 24.84 g (138 mmol) des Produktes in Form farbloser Kristallnadeln, entsprechend 99.3% der theoretisch möglichen Ausbeute. C₈H₈N₂O₃ (180 g/mol); berechnet: C: 53.33, H: 4.44, N: 15.55; gefunden: C: 52.41, H: 4.26, N: 14.68

### N-Tertiärbutyloxycarbonyl-4-carboxybenzoylhydrazid

20 g (0.11 mol) 4-Carboxybenzoylhydrazid werden in 200 ml THF suspendiert und mit einer Lösung von 23.98 g (0.11 mol) bis-*tert*.-Butyldicarbonat in 50 ml THF versetzt. Nach 48 h Rühren bei Raumtemperatur wird das THF i. Vak. entfernt, der ölige Rückstand in 400 ml Ethylacetat aufgenommen und 4 mal mit je 100 ml Wasser extrahiert. Nach Trocknen der organischen Phase über Na₂SO₄ wird auf die Hälfte eingeengt und auf 4 °C abgekühlt. Es bilden sich farblose Kristalle, die abgesaugt und mit Diethylether gewaschen werden. Man erhält 30.3 g (0.108 mmol) des Produktes entsprechend 98.4% der theoretisch möglichen

| | | | | |
|---|---|---|---|---|
| Ausbeute. C₁₃H₁₆N₂O₅ (280 g/mol) | berechnet | C: 55.71%, | H: 5.71%, | N: 10.00% |
| | gefunden | C: 56,94%, | H: 5.56%, | N: 9.79% |

### N-Tertiärbutyloxycarbonyl-4-(N-hydroxysuccinimidocarbonyl)benzoylhydrazid

4 g (14.29 mmol) N-Tertiärbutyloxycarbonyl-4-carboxybenzoylhydrazid werden zusammen mit 3.29 g (28.6 mmol) N-Hydroxysuccinimid und 17.4 mg DMAP in 100 ml THF gelöst und tropfenweise bei 4 °C mit einer Lösung von 3.23 g (15.72 mmol) DCC versetzt. Nach 12 h Rühren bei 4°C und 48 h bei Raumtemperatur wird das Lösungsmittel i. Vak. entfernt, der Rückstand in Ethylacetat gelöst und 10 mal mit je 50 ml gesättigter NaCl-Lösung extrahiert. Nach Trocknen der organischen Phase über Na₂SO₄ wird auf halbes Volumen eingeengt und auf 4°C abgekühlt. Es bilden sich farblose Nadeln, die abgesaugt und mit wenig Diethylether gewaschen werden. Man erhält 5.3 g (14.09 mmol), entsprechend 98.6% der theoretisch möglichen Ausbeute.

| | | | | |
|---|---|---|---|---|
| C₁₇H₁₈N₃O₇ (376 g/mol) | berechnet | C: 54.26%, | H: 4.79%, | N: 11.18% |
| | gefunden | C: 52.65%, | H: 5.53%, | N: 10.56% |

### 4-((N-Hydroxysuccinimido)carbonyl)benzoylhydrazid Trifluoracetat

3g (7.98 mmol) N-Tertiärbutyloxycarbonyl-4-(N-hydroxysuccinimidocarbonyl)benzoylhydrazid werden mit 10 ml wasserfreier Trifluoressigsäure versetzt und während 2 h bei Raumtemperatur gerührt. Unter starkem Rühren werden 50 ml Diethylether zugegeben. Der gebildete Feststoff wird abgesaugt und mehrmals mit Diethylether gewaschen. Nach Trocknen i. Vak. erhält man 3.0 g (7.67 mmol) des Produktes als weißes Pulver, entsprechend 96.1% der theoretisch möglichen Ausbeute. C₁₄H₁₂N₃O₇F₃ (391 g/mol) berechnet: C: 42.97%, H: 3.07%, N: 10.74%; gefunden: C: 43.49%, H: 3.42%, N: 10.74%

### Schritt 2: Synthese von Maleinimid- und Hydroxysuccinimidderivaten zytostatischer Verbindungen

### Chlorambucilcarbonsäurehydrazid

Chlorambucil (1,0 g; 3,29 mmol) wurde in 50 ml absoluten CH₂Cl₂ gelöst und mit Oxalylchlorid (431 µl; 4,8 mmol) versetzt und der Ansatz 15 h lang bei 34-40°C gerührt. Anschließend wurde die Lösung eingedampft und Reste von Oxalylchlorid im Hochvakuum entfernt. Das synthetisierte Oxalylcarbonsäurechlorid wurde unmittelbar weiter umgesetzt, indem der entstandene braune Sirup in 20 ml absoluten CH₂Cl₂ gelöst wurde und unter Rühren bei Raumtemperatur tert.-Butylcarbazat (457 mg; 3,45 mmol), gelöst in 20 ml CH₂Cl₂, innerhalb von 1 h zugetropft wurde. Der Ansatz wurde 36 h lang gerührt, vom Unlöslichen abfiltriert und die Lösung im Vakuum auf etwa 3 ml eingeengt. Das entstandene Chlorambucil-tert.-butylcarbazat wurde säulenchromatographisch gereinigt (Laufmittel: Essigester/Hexan 2/1; R_{f}-Wert: 0,52); Ausbeute: 700 mg (1,67 mmol; 51% d. Th.) brauner Sirup. Chlorambucil-tert.-butylcarbazat (700 mg; 1,67 mmol) wurde in 10 ml Tetrahydrofuran gelöst und unter Rühren bei Raumtemperatur mit 10 ml Trifluoressigsäure versetzt, 1 h lang gerührt und anschließende das Lösungsmittel und die Trifluoressigsäure im Hochvakuum entfernt unter Bildung eines hellbraunen Feststoffes.

### Carboxylhydrazonderivat von [4-(4-bis(2-chlorethyl)aminophenyl)]buttersäurehydrazid (Trifluoracetat-Salz) und 4-Acetophenoncarbonsäure-(N-hydroxysuccinimid)-ester

Chlorambucilcarbonsäurehydrazid (Trifluoracetat-Salz; 721 mg; 1,67 mmol) wurde in 30 ml Tetrahydrofuran gelöst und unter Rühren bei Raumtemperatur mit 4-Acetophenoncarbonsäure-(N-hydroxysuccinimid)-ester (479 mg; 1,83 mmol) versetzt. Nach 20 Minuten wurde die Reaktionslösung eingedampft. Der Rückstand wurde aus Essigester/Hexan umkristallisiert; Ausbeute: 290 mg (0,66 mmol; 40 % d. Th.) hellgelbe Kristalle; (C₂₇H₃₁N₄O₆Cl₂, Mr 577), berechnet: C 56,2 % H 5,4 % N 9,7 % Cl 12,1 %; gefunden: C 56,3 % H 5,6 % N 12,4 %, Cl 11,7%.

### N¹-(2-Maleinimidoethyloxycarbonyl)-5-fluorouracil

500 mg (3,84 mmol) 5-Fluorouracil wurden in 100 ml Tetrahydrofuran gelöst und mit 505 mg (696 µl; 4,99 mmol) Triethylamin versetzt. Zu dieser Lösung wurden 1016 mg (4,99 mmol) Chlorameisensäure-2-maleinimidoethylester, gelöst in 100 ml Tetrahydrofuran, zugetropft und der Ansatz 15 h lang bei Raumtemperatur gerührt. Anschließend wurde das Lösungsmittel im Wasserstrahlvakuum entfernt und der Rückstand säulenchromatographisch gereinigt (LOBAR®-säule; Laufmittel: Essigester/ Hexan 1,5/1; Ausbeute: 61 %); Schmelzpunkt: 132°C; R_{f}-Wert: 0,64 (Diol; Essigester/Hexan 2/1) berechnet: C: 44,29 % H: 2.68 % N: 14,09 % (C₁₁H₈N₃O₆F); gefunden: C: 44,29 % H: 2,68 % N: 13,58 %

### N¹-(m-Maleinimidobenzoyloxymethyl)-5-fluorouracil

1500 mg (11,55 mmol) 5-Fluorouracil und 2,25 ml (25,4 mmol) 37 %ige Formaldehydlösung wurden 2 h lang unter Rühren auf 60°C erwärmt. Anschließend wurde das Wasser im Hochvakuum entfernt und der Rückstand unter Zugabe von 20 mg DMAP in 80 ml Tetrahydrofuran gelöst. Zu dieser Lösung wurden 3,01 g (13,85 mmol) m-Maleinimidobenzoesäure und 2,858 g (13,85) DCC, gelöst in 50 ml Tetrahydrofuran, zugegeben und anschließend 15 h lang bei Raumtemperatur gerührt. Der Niederschlag wurde abfiltriert und das Lösungsmittel im Vakuum entfernt. Der Rückstand wurde in etwa 20 ml Essigester aufgenommen und vom Ungelösten abfiltriert. Die Lösung wurde eingedampft und der Rückstand chromatographiert (1. Kieselgel (Essigester/Hexan 2/1); Ausbeute: 5 % weißes Pulver); Schmelzpunkt: Zersetzung >250°C; R_{f}-Wert: 0,32 (Essigester/Hexan 2/1).

| | | | | |
|---|---|---|---|---|
| Elementaranalyse: (C₁₆H₁₀N₃O₆F) | berechnet | C: 55,98 % | H: 2,92 % | N: 12,24 % |
| | gefunden | C: 55,51 % | H: 2,87 % | N: 11,72 % |

### 3'-Aminoamidderivat von Doxorubicin mit p-Maleinimidophenylessigsäurechlorid

500 mg (0.86 mmol) Doxorubicin Hydrochlorid wurden in 50 ml absolutem DMF gelöst und 1013 mg (4.30 mmol)*p*-Maleinimidophenylessigsäurechlorid und 719 µl (522 mg; 5.16 mmol) Triethylamin zugegeben. Die Lösung wurde bei Raumtemperatur 15 h lang gerührt. DMF unter Hochvakuum entfernt und der Rückstand in 5 ml Tetrahydrofuran gelöst, filtriert und über eine Kieselgelsäule (Tetrahydrofuran/Hexan 3/1) gereinigt: 189 mg des roten Produktes (29 %); R_{f}-Wert: 0.26 (Ethylacetat/Hexan 3/1); Schmelzpunkt: 110 °C; C₃₉H₃₆N₂O₁₄: berechnet: C 61,84 % H 4.75 % N 3,70 %. gefunden: C 61,35 % H 5,14 % N 3,45 %.

### C-13 Benzoylhydrazonderivat von Doxorubicin und 4-((N-Hydroxysuccinimido) carbonyl)benzoylhydrazid Trifluoracetat

0.2 mmol Doxorubicin Hydrochlorid und 1.0 mmol 4-((N-Hydroxysuccinimido)carbonyl)benzoylhydrazid (Trifluoroacetat-Salz) wurden in 100 ml absoluten Methanol gelöst. Zu dieser Lösung wurden 100 µl CF₃COOH zugegeben und die Reaktionsmischung 36 h lang bei Raumtemperatur gerührt. Die Lösung wurde anschließend auf etwa 50 ml eingeengt. Acetonitril wurde bis zur Trübung zugegeben und die Suspension bei -20 °C 24 h lang kaltgestellt. Das Produkt wurde durch Zentrifugieren gesammelt und aus Methanol/Acetonitril umkristallisiert: 276 mg, R_{f}-Wert (reverse phase, Acetonitril/0.005 M NaH₂PO₄ (pH 5.0) = 70/30): 0.33, Schmelzpunkt: > 200 °C (Zersetzung), C₃₉H₄₀N₄O₁₄Cl: berechnet: C 56,83 % H 4,86 % N 6,80 % Cl 4,31 %; gefunden: C 57.04 % H 5,14 % N 6,55 % Cl 4,12 %.

Das Verfahren für die Herstellung der Maleinimidderivaten mit cis-konfigurierten Platin(II)-Einheiten wird durch folgendes Beispiel erläutert: N-(O-(3-Maleinimidobenzoyl)-2-hydroxyethyl)-1,2-diaminoethandichloroplatin(II) - vierstufige Synthese.

### N-(2-Hydroxyethyl)-N,N'-bis-tertiärbutyloxycarbonyl-1,2-diaminoethan

Zu einer Lösung von 20.8 g (200 mmol) N-(2-Hydroxyethyl)-1,2-Diaminoethan in 100 ml Dichlormethan werden 47.96 g (220 mmol, 0.5 eq) bis-Tertiärbutyloxycarbonylanhydrid, gelöst in 200 ml Dichlormethan innerhalb von 1 h bei Raumtemperatur zugetropft. Der Ansatz wird für 12 h bei Raumtemperatur gerührt, dann mit 100 ml Diethylether verdünnt und mit 150 ml Wasser extrahiert. Nach dem Trocknen der organischen Phase über Natriumsulfat wird i. Vak. eingeengt. Die Reinigung des Rohproduktes erfolgt durch Säulenchromatographie (Ethylacetat/Hexan (1:1,5), R_{f}-Wert (Ethylacetat/Hexan 1:1,5): R_{f} = 0.18; Ausbeute: 30 g (98.68 mmol). Elementaranalyse für C₁₄H₂₈N₂O₅ (304 g/mol); berechnet: C: 55.26%, H: 9.21%, N: 9.21%; gefunden: C: 55.50%, H: 9.18%, N: 9.02

### N-(O-(3-Maleinimidobenzoyl)-2-hydroxyethyl)-N,N'-bis-tertiärbutyloxycarbonyl-1,2-diaminoethan

Zu einer Lösung von 8 g (26.3 mmol) N-(2-Hydroxyethyl)-N,N'-bis-tertiärbutyloxycarbonyl-1,2-diaminoethan in 50 ml Tetrahydrofuran und 4 ml (28.9 mmol, 1.1 eq) Triethylamin werden 6.82 g (29 mmol, 1.1 eq) Maleinimidobenzoesäurechlorid, gelöst in 100 ml Tetrahydrofuran, bei Raumtemperatur unter Rühren innerhalb 1 h zugetropft. Nach weiteren 8 h Rühren bei Raumtemperatur ist laut DC vollständiger Umsatz erreicht. Das bei der Reaktion gebildete Triethylammoniumchlorid wird abfiltriert. Nach Entfernen von Tetrahydrofuran und überschüssigem Triethylamin i. Vak. wird das anfallende Öl säulenchromatographisch gereinigt (Kieselgel: Ethylacetat/Hexan (1:1), R_{f}-Wert (Ethylacetat/Hexan 1:1) = 0.2, Ausbeute: 9.6 g (19.1 mmol) des Produkts in Form eines gelben Öls, entsprechend 72.6% der theoretisch möglichen Ausbeute. Elementaranalyse für C₂₅H₃₃N₃O₈ (503 g/mol): berechnet: C: 59.64%, H: 6.56%, N: 8.35%; gefunden: C: 60.04%, H: 6.77%, N: 8.24%

### N-(O-(3-Maleinimidobenzoyl)-2-hydroxyethyl)-1,2-diaminoethan Dihydrochlorid

6 g (11.93 mmol) N-(O-(3-Maleinimidobenzoyl)-2-hydroxyethyl)-N,N-bis-tertiärbutyloxy-carbonyl-1,2-diaminoethan werden mit 60 ml (5 eq) einer 1M Lösung von HCl in Diethylether unter Rühren bei Raumtemperatur versetzt. Nach 48 h Rühren bei Raumtemperatur wird der feinkristalline Niederschlag über eine G4-Glasfritte abgesaugt, durch mehrmaliges Waschen mit wasserfreiem Ether von HCl-Resten befreit und i. Vak. getrocknet. Man erhält 3,0 g (7.98 mmol) des Produkts als feinpudrigen, gelben Feststoff, entsprechend 66.9% der theoretisch möglichen Ausbeute. Elementaranalyse für C₁₅H₁₉N₃O₄Cl₂ (375.9 g/mol)

| | | | | |
|---|---|---|---|---|
| berechnet | C: 47.89%, | H: 5.05%, | N: 11.17%, | Cl: 18.86% |
| gefunden | C: 46.97%, | H: 5.42%, | N: 10.03%, | Cl: 17.63% |

### N-(O-(3-Maleinimidobenzoyl)-2-hydroxyethyl)-1,2-diaminoethandichloroplatin(II)

104 mg (0.25 mmol) K₂PtCl₄ und 94.2 mg (0.25 mmol) N-(O-(3-Maleinimidobenzoyl)-2-hydroxyethyl)-1,2-diaminoethan Dihydrochlorid werden in jeweils 5 ml 20% Tetrahydrofuran/Wasser gelöst und portionsweise vermischt. Die zunichst rote K₂PtCl₄-Lösung wird zunehmend entfärbt, es bildet sich ein hellgelber Niederschlag. Etwa 1 h nach der letzten Zugabe kann das Präzipitat durch eine G4-Glasfritte abgesaugt werden. Es wird sukzessive mit kleinen Portionen Wasser, 20% Tetrahydrofuran/Wasser und zuletzt mit Diethylether gewaschen. Nach dem Trocknen i. Vak. erhält man 106 mg (0.18 mmol) des Produktes als feinkristalline Substanz, entsprechend 72% der theoretisch möglichen Ausbeute. Elementaranalyse für C₁₅H₁₇N₃O₄PtCl₂ (569 g/mol); berechnet: C: 31.63%, H: 2.99%, N: 7.38%, Pt: 34.29%, Cl: 12.46%; gefunden: C: 31.19%, H: 3.37%. N: 6.79%, Pt: 32.35%, Cl: 12.95%

### Schritt 3: Thiolierung des Trägerproteins

Das Verfahren für die Thiolierung wird durch folgendes Beispiel genauer dargestellt: 28,4 mg humanes Serumalbumin (98 % kristallin, Mr 66500)] werden in 1 ml mit Argon entgastern Puffer (0,1 M Natriumborat, 0.001M EDTA, 0,15 M NaCl - pH = 8,0) gelöst (c(Albumin) = 4,0 x 10⁻⁴ M) gelöst und mit 100 µl einer frisch hergestellten 4 x 10⁻² M Iminothiolanlösung (5,5 mg Iminothiolan gelöst in 1 ml entgastem Puffer (0,1 M Natriumborat, 0,15 M NaCl, 0.001M EDTA - pH = 8,0) versetzt. Nach 60-70 min wird das überschüssige Iminothiolan durch Gelfiltration (Säule 1,0 cm x 10 cm, Sephadex G.25) mit dem Laufpuffer 0.05 M Natriumborat, 0.15M NaCl - pH 7.5 vom thiolierten Albumin getrennt. Die Proteinkonzentration nach erfolgter Gelfiltration wurde bei 280 nm, ε₂₈₀ = 35 700 M⁻¹ cm⁻¹, (c[Albumin] = 2,2 x 10⁻⁴ M) und die Anzahl der eingeführten HS-Gruppen mit Ellmanns Reagenz bei 412 nm ε₄₁₂ = 13 600 M⁻¹ cm⁻¹) bestimmt (c[HS-Gruppen] = 7,7 x 10⁻⁴ M). Das Verhältnis c[HS-Gruppen]/c[Albumin] betrug 3,5. Die folgenden Tabelle faβt die Reaktionsbedingungen zusammen, durch die eine unterschiedliche Anzahl von HS-Gruppen in Albumin eingeführt werden:

**Tabelle 1.**

| **Reaktionsbedingunge für die Einführung von HS-Gruppen in Albumin** | | | |
|---|---|---|---|
| c[Iminothiolan]/ c[Protein] | Reaktionszeit, min | Temperatur, °C | Anzahl eingeführter HS-Gruppen/Protein |
| 10:1 | 60-75 | 0-5 | 1 |
| 10:1 | 60-75 | 20-25 | 2-3 |
| 20:1 | 60-73 | 20-25 | 5-6 |
| 30:1 | 60-75 | 20-25 | 9-10 |
| 50:1 | 60-95 | 20-25 | 15-16 |
| 100:1 | 60-90 | 20-25 | 30-32 |

Die so thiolierte Proteinprobe wurde direkt für die folgende Reaktion im Schritt 4 verwendet.

### Schritt 4: Kopplung der Derivate der zytostatischen Verbindungen (Schritt 2) an das thiolierte Trägerprotein (Schritt 3):

*Methoden-* FPLC für die Herstellung der Konjugate: P-500 pump, LCC 501 Controller (Pharmacia) und LKB 2151 UV-Monitor, Puffer: Standardborat: 0.025 M Natriumborat, 0.15 M NaCl - pH 7.5 oder Phosphatpuffer: 0.004 M Natriumphosphat, 0.15 M NaCl - pH 7.4. Die Proteinkonzentration des Konjugats wurde mit dem BCA-Protein-Essay von Pierce (USA) bestimmt

### Albuminkonjugat des Carboxylhydrazonderivat von [4-(4-bis(2-chlorethyl)aminophenyl)]buttersäurehydrazid und 4-Acetophenoncarbonsäure-(N-hydroxysuccinimid)-ester

66,5 mg Albumin, gelöst in 2,2 ml 5 ml Phosphatpuffer, wurden mit 0,1 ml einer Lösung des Carboxylhydrazonderivat in DMF (1,2 mg gelöst in 0,1 ml DMF) vermischt, nach 10 min zentrifugiert und der Überstand auf eine Sephadex® G-25F Säule (Säule 1,0 cm x 10 cm) gegeben und das Konjugat isoliert (Retentionsvolumen: 3,7-7,1 ml). Die Menge an gebundenem Chlorambucil wurde mit Hilfe des Tests nach Epstein (Epstein et al., *Anal. Chem.* 1955, 27, 1435-1439) bestimmt. Sie betrug bei diesem Arbeitsvorgang 280 µM.

### Konjugat bestehend aus mit Doxorubicin (Amid₁) beladenem Albumin und Transferrin

30 mg Humansemmalbumin wurden in 2,0 ml mit Argon entgastem 0,1 M Natriumborat, 0,001 M EDTA, 0.15 M NaCl - pH = 8,0 bei Raumtemperatur (RT) gelöst. Zu dieser Mischung wurden 183 µl einer Lösung aus 2,9 mg Iminothiolan in 250µl entgastern 0,1 M Natriumborat, 0.001 M EDTA, 0,15 M NaCl - pH = 8,0 zugegeben, gut durchmischt und 1 h bei RT inkubiert. Anschließend wurde überschüssiges Iminothiolan durch Gelfiltration abgetrennt. Die Messung der Proteinkonzentration, sowie der Konzentration an freien SH-Gruppen ergab folgende Werte: c[Albumin] = 1.44x10⁻⁴ mol/l, c[SH-Gruppen] = 1.25x10⁻³ mol/l. Die durchschnittliche Zahl der SH-Gruppen pro Albuminmolekül ergab sich aus dem Quotienten beider Konzentrationen zu 8.7.

2,0 ml dieser thiolierten Albuminlösung wurden für die weitere Umsetzung verwendet. Um 7 der 8,7 SH-Gruppen pro Protein mit 3'-Aminoamidderivat von Doxorubicin und p-Maleinimidophenylessigsäure (Amid₁) abzusättigen wurde die Eiweißlösung zunächst mit Standardboratbuffer auf 10 ml verdünnt. Dann wurden unter Schütteln 40µl einer Lösung von 40 mg Amid₁ in 1 ml DMF zugegeben und 10 Minuten lang bei RT inkubiert. Danach wurde die Probe 5 min lang zentrifugiert (3000 g, 4 °C). Der Überstand wurde mit Centricon 3000-Konzentratoren® (Fa. Amicon) auf ein Volumen von etwa 1 ml eingeengt (3000g, 3x15 min, 4 °C). Anschließend wurden 25 mg Humanserumtransferrin in 1,2 ml in mit Argon entgastern Puffer (0,1 M Natriumborat, 0,001 M EDTA, 0,15 M NaCl - pH = 8,0) gelöst. Zu dieser Mischung wurden 15 µl einer Lösung aus 2,9 mg iminothiolan in 250µl in mit Argon entgastern Puffer (0,1 M Natriumborat, 0,001 M EDTA, 0,15 M NaCl - pH = 8,0) zugegeben, durchmischt und 1 h lang bei RT inkubiert. Anschließend wurde überschüssiges Iminothiolan durch Gelfiltration abgetrennt. Die Messung der Proteinkonzentration sowie der Konzentration an freien SH-Gruppen (Test nach Ellmann) ergab folgende Werte: [Transferrin] = 1,20x10⁻⁴ mol/l, [SH-Gruppen] = 1,96x10⁻⁴ mol/l. Die durchschnittliche Zahl der SH-Gruppen pro Transferrinmolekül ergab sich aus dem Quotienten beider Konzentrationen zu 1,6.

1,9 ml der so thiolierten Transferrinlösung wurden für die weitere Umsetzung zunächst mit Standardboratpuffer auf 10 ml verdünnt. Dann wurden der Reaktionsmischung 72 µl einer Lösung von 10 mg 1,4-Diamino-N,N'-di-m-maleinimidobenzyl-butan in 200 µl DMF zugegeben. Nach 10 min bei RT wurde die trübe Mischung zentrifugiert (5 min, 3000 g, 4 °C). Die überstehende Lösung wurde abdekantiert und in einem Centricon®3000 Konzentrator auf ein Volumen von 600 µl eingeengt. Zur Entfernung überschüssigen Bismaleinimid wurde über Sephadex 25 gefiltriert.

1500 µl einer Lösung mit dem so modifizierten Transferrin wurden zu 500 µl der obigen mit Doxorubicin beladenen Albuminlösung gegeben, gut vermischt und 15 min lang bei RT inkubiert. Anschließend wurde die Lösung in Microcon 10 Konzentratoren® (Fa. Amicon) auf ein Volumen von 150 µl eingeengt. Die konzentrierte Eiweißlösung wurde gelchromatographisch in ihre Bestandteile (Monomere, Dimere, Oligomere) getrennt. Säulendimension: h: 40 cm, Ø: 1 cm, Loop: 100 µl, stationäre Phase: Superdex 200 Pharmacia, mobile Phase: Boratpuffer; pH 6,8; +4 °C N₂- begast, Fluß: 1 ml/min. Detektion: photometrisch, λ= 280 nm, Retentionsvolumina: Oligomere: 9.5 ml - 11.5 ml, Trimere: 11,7 ml -12,6 ml, Dimere: 12,7 ml - 14,4 ml, Monomere: 14,5 ml - 18,5 ml

Die Ausbeute der gewünschten Dimere bezüglich des eingesetzten Amid, betrug 20 - 30 %.

### Biologische Untersuchungen

Als Beispiel für die *in vitro* und *in vivo* Wirksamkeit der Konjugate werden die biologischen Daten der unten abgebildeten Doxorubicin-Konjugate aufgeführt, die repräsentativ für das Aktivitäts- bzw. Toxizitätsprofil der in der Erfindung offenbarten Konjugate sind. Die Wirksamkeit der Konjugate wurde mit einem "colony forming assay" und mit Hilfe des BrdU(5-Brom-2'-desoxyuridin)-Einbaus gemäß wissenschaftlicher Gepflogenheit in der Zellkultur bestimmt. Als Beispiele sind die IC₇₀-Werte ("colony forming assay" - sieben Xenografts) folgender Doxorubicin-Konjugate aufgezeigt: Protein = Albumin (A)

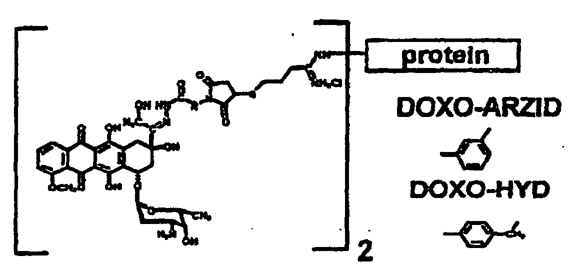

| **IC70-Werte in sieben Human-Tumor-Xenografts (colony forming assay)** | | | |
|---|---|---|---|
| Human-Tumor-Xenograft | DOXORUBICIN | A-DOXO-ARZID | A-DOXO-HYD |
| bladder BXF1299 | 0.50 | 0.06 | 0.10 |
| lung LXFL 529 | 0.02 | 0.03 | 0.04 |
| lung LXFS 538 | 0.02 | 0.001 | 0.01 |
| mamma MAXFMX1 | 0.02 | 0.01 | 0.10 |
| melanoma MEXF989 | 0.30 | 0.34 | 0.30 |
| prostate PC3MX | 0.03 | 0.02 | 0.06 |
| prostate DU145X | 0.35 | 0.24 | 0.21 |

Unter diesen experimentellen Bedingungen zeigten die wie vorstehend aufgeführt synthetisierten Konjugate eine der ungebundenen zytostatischen Verbindung gleichwerüge bzw. höhere Wirksamkeit.

Des weiteren zeigen die oben abgebildeten Konjugate im Vergleich zum freien Doxorubicin in den xenotransplantierbaren Nacktmausmodellen Mammacarcinoma MDA-MB-435 und Mammacarcinoma MCF-7 insgesamt eine gegenüber dem freien Doxorubicin deutlich verringerte Toxizität (verringerte Letalität und Körpergewichtsreduktion. weniger Nebenwirkungen im Gastrointestinal-Bereich) und eine Stabilisierung der relativen Tumorvolumenzunahme bei gleicher bzw. verbesserter tumorhemmender Wirksamkeit auf.

## Patentansprüche

1. Konjugat einer zytostatischen Verbindung und Albumin , wobei
2 bis 30 Äquivalente der zytostatischen Verbindung jeweils über einen Spacer, der eine von einer Maleinimidogruppe stammende Gruppe umfaßt, an thioliertes Albumin gekoppelt sind, das über eine von einer Bismaleinimidoverbindung stammende Gruppe an Transferrin oder einen gegen ein tumorassoziiertes Antigen gerichteten monoklonalen Antikörper konjugiert ist,
und wobei das thiolierte Albumin im Mittel 1 bis 30 HS-Gruppen aufweist.

2. Konjugat nach Anspruch 1, wobei die zytostatische Verbindung aus der Gruppe der Anthrazykline, der Stickstofflostderivate, der Purin- oder Pyrimidinantagonisten, der Folsäureantagonisten, der Taxoide, der Camptothecine, der Podophyllotoxinderivate, der Vinca-Alkaloide oder der *cis*-konfigurierten Platin(II)-Komplexe ausgewählt ist.

3. Konjugat nach Anspruch 1 oder 2, wobei die zytostatische Verbindung aus der Gruppe, bestehend aus Doxorubicin, Daunorubicin, Epirubicin, Idarubicin, Mitoxantron, Chlorambucil, Melphalan, 5-Fluorouracil, 5'-Desoxy-5-fluorouridin, Thioguanin, Methotrexat, Paclitaxel, Docetaxel, Topotecan, 9-Aminocamptothecin, Etoposid, Teniposid, Mitopodozid, Vinblastin, Vincristin, Vindesin, Vinorelbin und Verbindungen der allgemeinen Formel I, II, III oder IV, 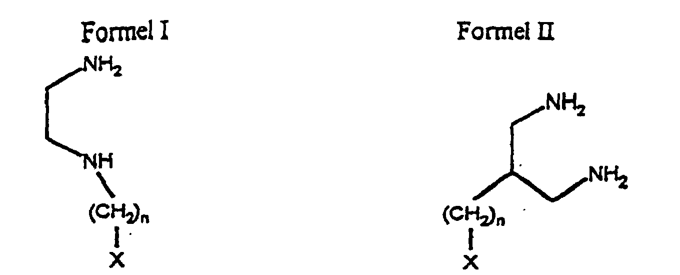 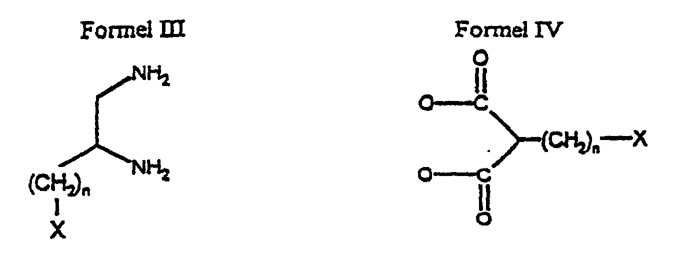 ausgewählt ist, wobei
n = 0-6, X = -NH₂, -OH, -COOH, -O-CO-R-COR* oder -NH-CO-R-COR* ist, worin R eine aliphatische Kohlenstoffkette mit 1-6 Kohlenstoffatomen oder eine substituierte bzw. unsubstituierte Phenylengruppe ist, und R* H, Phenyl oder Alkyl mit 1-6 Kohlenstoffatomen bedeutet.

4. Konjugat nach Anspruch 3, wobei die zytostatische Verbindung mit Spacer, der eine von einer Maleinimidogruppe stammende Gruppe umfaßt, durch Umsetzung der zytostatischen Verbindung mit einer Maleinimidverbindung der Formel V, VI oder VII 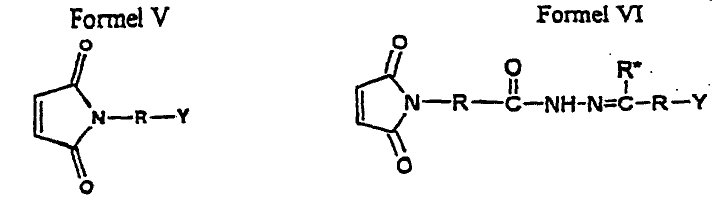 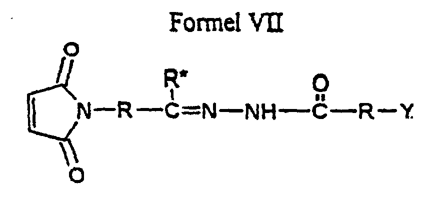 worin für den Fall, daß R eine aliphatische Kohlenstoffkette mit 1-6 Kohlenstoffatomen ist, Y = -OH; -COOH, -COCl, -CONH-(CH₂)ₙ-OH, -COO-(CH₂)ₙ-NH₂, -COO-(CH₂)ₙ-NHNH₂, -SO₃H, -SO₃Cl, -SO₂-NHNH₂, -O-COCl, -CHO oder -COR* mit n = 1-6 ist und R* H, Phenyl oder Alkyl mit 1-6 Kohlenstoffatomen bedeutet, und worin für den Fall, daß R eine substituierte bzw. unsubstituierte Benzylgruppe oder eine substituierte bzw. unsubstituierte Phenylengruppe ist, Y = -OH, -COOH, -COCl, -CONH-(CH₂)ₙ-OH, -COO-(CH₂)ₙ-NH₂, -COO-(CH₂)ₙ-NHNH₂, -SO₃H, -SO₃Cl, -SO₂-NHNH₂, -O-COCl, -CHO, -COR* oder -CO-NHNH₂ mit n = 1-6 ist und R* H, Phenyl oder Alkyl mit 1-6 Kohlenstoffatomen bedeutet, entsteht, und das so erhaltene Maleinimidderivat der zytostatischen Verbindung an das thiolierte Albumin gekoppelt wird, wobei die chemische Verknüpfung zwischen zytostatischer Verbindung und Maleinimidverbindung durch eine Amid-, Ester-, Imin-, Hydrazon-, Carboxylhydrazon-, Oxycarbonyl-, Acetal- oder Ketalbindung erfolgt.

5. Konjugat nach Anspruch 3 oder 4, wobei die von einer Bismaleinimidoverbindung stammende Gruppe durch Konjugieren des mit 2 bis 30 Äquivalenten der zytostatischen Verbindung gekoppelten thiolierten Albumins mit einer Bismaleinimidverbindung der Formel XI 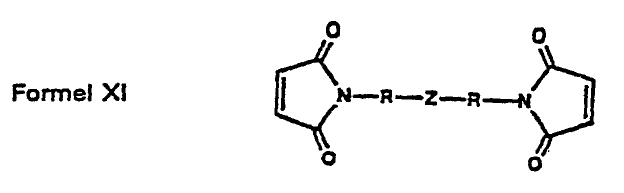 entsteht, worin Z = -CO-NH-(CH₂)ₙ-NH-CO-, -CO-O-(CH₂)ₙ-O-CO-, -CH = N-(CH₂)ₙ-N = CH-, -CH = N-NH-(CH₂)ₙ-NH-N = CH- oder -CH = N-NH-CO-(CH₂)ₙ-CO-NH-N = CH- und n = 2-12 ist.

6. Verfahren zur Herstellung eines Konjugats einer zytostatischen Verbindung und Albumin nach einem der Ansprüche 1 bis 5, umfassend die Schritte:
(a) Umsetzen einer zytostatischen Verbindung mit einer Maleinimidverbindung, so daß Maleinimidderivate der zytostatischen Verbindung hergestellt werden, wobei die chemische Verknüpfung zwischen zytostatischer Verbindung und Maleinimidverbindung durch eine Amid-, Ester-, Imin-, Hydrazon-, Carboxylhydrazon-, Oxycarbonyl-, Acetal- oder Ketalbindung erfolgt, und
(b) Beladen von thioliertem Albumin mit 2 bis 30 Äquivalenten des im Schritt (a) erhaltenen Maleinimidderivats der zytostatischen Verbindung und Konjugieren mit Transferrin oder einem gegen ein tumorassoziiertes Antigen gerichteten monoklonalen Antikörper über eine von einer Bismaleinimidoverbindung stammenden Gruppe, wobei das thiolierte Albumin im Mittel 1 bis 30 HS-Gruppen aufwrist

7. Arzneimittel, enthaltend ein Konjugat nach einem der Ansprüche 1 bis 5, gegebenenfalls mit üblichen Trägern und Hilfsstoffen.

8. Verwendung eines Konjugats nach einem der Ansprüche 1 bis 5 zur Herstellung eines Arzneimittels zur Behandlung von Krebskrankheiten.

9. Verwendung nach Anspruch 8, wobei die Krebskrankheiten Blasen-, Lungen-, Mamma-, Melanom- und Prostatakarzinome umfassen.

## Claims

1. A conjugate of a cytostatic compound and albumin, wherein 2 to 30 equivalents of said cytostatic compound are each coupled via a spacer comprising a group which is derived from a maleinimido group, to thiolated albumin which is conjugated to transferrin or a monoclonal antibody which is directed to a tumor-associated antigen, via a group which is derived from a bismaleinimido compound, said thiolated albumin having 1 to 30 HS groups on the average.

2. The conjugate according to claim 1, wherein said cytostatic compound is selected from the group consisting of anthracyclines, nitrogen mustard derivatives, purine or pyrimidine antagonists, folic acid antagonists, taxoids, camptothecines, podophyllotoxin derivatives, vinca alkaloids and cis-configured platinum(II)-complexes.

3. The conjugate according to claim 1 or 2, wherein said cytostatic compound is selected from the group consisting of doxorubicine, daunorubicine, epirubicine, idarubicine, mitoxantrone, chlorambucil, melphalan, 5-fluorouracil, 5'-deoxy-5-fluorouridine, thioguanine, methotrexate, paclitaxel, docetaxel, topotecane, 9-aminocamptothecine, etoposide, teniposide, mitopodizide, vinblastine, vincristine, vindesine, vinorelbine and compounds of the general formulas I, II, III, or IV: 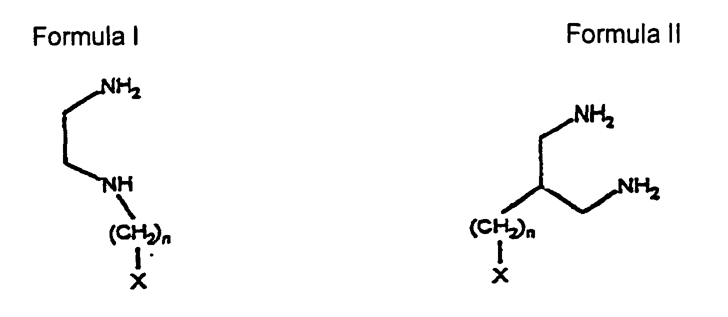 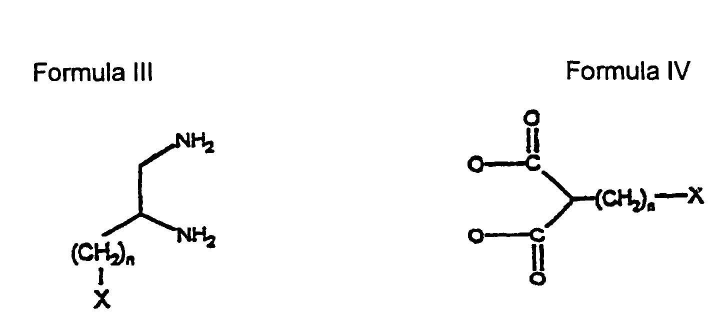 wherein n = 0-6, X = -NH₂, -OH, -COOH, -O-CO-R-COR* or -NH-CO-R-COR*, wherein R is an aliphatic carbon chain having 1-6 carbon atoms or is a substituted or unsubstituted phenylene group, and R* is H, phenyl or alkyl having 1-6 carbon atoms.

4. The conjugate according to claim 3, wherein said cytostatic compound having said spacer comprising a group which is derived from a maleinimido group, is formed through reaction of said cytostatic compound with a maleinimide compound of the formula V, VI or VII: 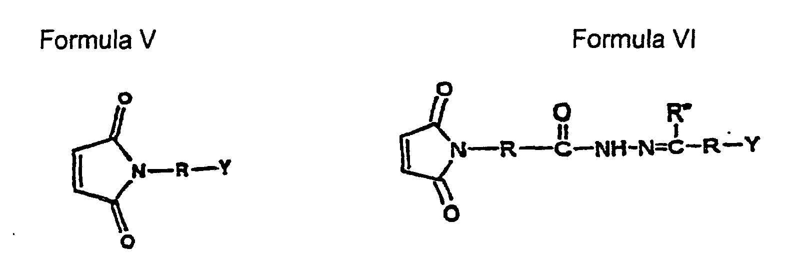 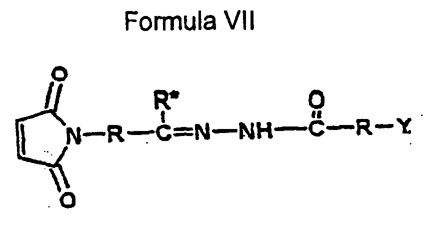 wherein in case R is an aliphatic carbon chain having 1-6 carbon atoms, Y = -OH, -COOH, -COCl, -CONH-(CH₂)ₙ-OH, -COO-(CH₂)ₙ-NH₂, -COO-(CH₂)ₙ-NHNH₂, -SO₃H, -SO₃Cl, -SO₂-NHNH₂, -O-COCl, -CHO or -COR*, wherein n = 1-6 and R* represents H, phenyl or alkyl having 1-6 carbon atoms, and wherein in case R is a substituted or unsubstituted benzyl group or a substituted or unsubstituted phenyl group, Y = -OH, -COOH, -COCl, -CONH-(CH₂)ₙ-OH, -COO-(CH₂)ₙ-NH₂, -COO-(CH₂)ₙ-NHNH₂, -SO₃H, -SO₃Cl, -SO₂-NHNH₂, -O-COCl, -CHO, -COR* or -CO-NHNH₂, wherein n = 1-6 and R* represents H, phenyl or alkyl having 1-6 carbon atoms, and the thus obtained maleinimide derivative of said cytostatic compound is coupled to said thiolated albumin, wherein the chemical linkage between said cytostatic compound and said maleinimide compound occurs through an amide, ester, imine, hydrazone, carboxyl hydrazone, oxycarbonyl, acetal or ketal bond.

5. The conjugate according to claim 3 or 4, wherein said group which is derived from a bismaleinimido compound, is formed through conjugating said thiolated albumin, which is coupled to 2 to 30 equivalents of said cytostatic compound, with a bismaleinimide compound of the formula XI: 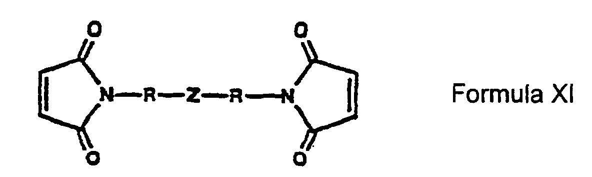 wherein Z = -CO-NH-(CH₂)ₙ-NH-CO-, -CO-O-(CH₂)ₙ-O-CO-, -CH=N-(CH₂)ₙ-N=CH-, -CH=N-NH-(CH₂)ₙ-NH-N=CH- or -CH=N-NH-CO-(CH₂)ₙ-CO-NH-N=CH- and n = 2-12.

6. A method for producing a conjugate of a cytostatic compound and albumin according to anyone of claims 1 to 5, comprising the steps of:
(a) reacting a cytostatic compound with a maleinimide compound, such that maleinimide derivatives of said cytostatic compound are produced, wherein the chemical linkage between said cytostatic compound and said maleinimide compound occurs through an amide, ester, imine, hydrazone, carboxyl hydrazone, oxycarbonyl, acetal or ketal bond; and
(b) loading thiolated albumin with 2 to 30 equivalents of said maleinimide derivative of the cytostatic compound obtained in step (a) and conjugating with transferrin or a monoclonal antibody directed to a tumor-associated antigen via a group which is derived from a bismaleinimido compound, said thiolated albumin having 1 to 30 HS groups on the average.

7. A pharmaceutical composition containing a conjugate according to anyone of claims 1 to 5, optionally together with conventional carriers and auxiliary agents.

8. Use of a conjugate according to any one of claims 1 to 5 for the manufacture of a pharmaceutical composition for treating cancer diseases.

9. The use according to claim 8, wherein said cancer diseases comprise bladder, lung, mamma, melanoma and prostate carcinomas.

## Revendications

1. Conjugué d'un composé cytostatique et d'albumine, où 2 à 30 équivalents du composé cytostatique sont couplés par le biais d'un écarteur, qui comprend un groupe provenant d'un groupe maléinimido, à une albumine thiolée, qui est conjuguée par le biais d'un groupe provenant d'un composé bismaléinimido à une transferrine ou à un anticorps monoclonal dirigé contre un antigène associé aux tumeurs, et où l'albumine thiolée comprend en moyenne 1 à 30 groupes HS.

2. Conjugué selon la revendication 1, où le composé cytostatique est choisi dans le groupe des anthracyclines, des dérivés non azotés, des antagonistes de purine ou de pyrimidine, des antagonistes d'acide folique, des taxoïdes, des camptothécines, des dérivés de podophyllotoxine, des alcaloïdes de Vinca ou des complexes de platine (II) configurés cis.

3. Conjugué selon la revendication 1 ou 2, où le composé cytostatique est choisi dans le groupe constitué de la doxorubicine, la daunorubicine, l'épirubicine, l'idarubicine, la mitoxantrone, le chlorambucil, le melphalan, le 5-fluorouracil, la 5'-désoxy-5-fluorouridine, la thioguanine, le méthotrexate, le paclitaxel, le docétaxel, le topotecan, la 9-aminocamptothécine, l'étoposide, le téniposide, le mitopodozide, la vinblastine, la vincristine, la vindésine, la vinorelbine et les composés des formules générales I, II, III ou IV, 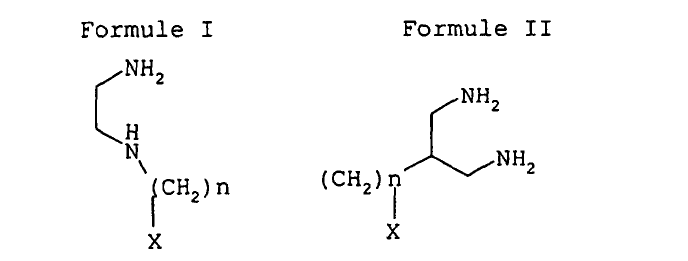 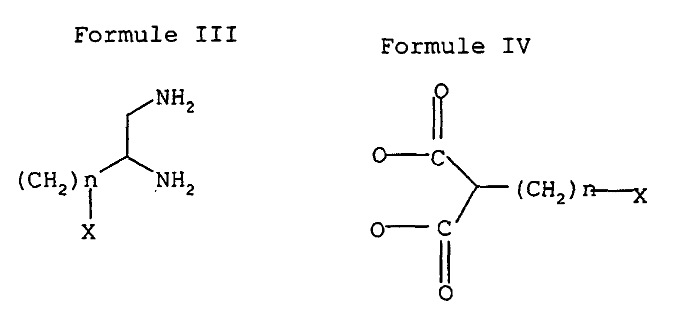 dans lesquelles n = 0-6, X = -NH₂, -OH, -COOH, -O-CO-R-COR* ou -NH-CO-R-COR*, où R est une chaîne carbonée aliphatique ayant de 1 à 6 atomes de carbone ou un groupe phénylène substitué ou non substitué et R* est H, un groupe phényle ou alkyle avec 1 à 6 atomes de carbone.

4. Conjugué selon la revendication 3, où le composé cytostatique avec l'écarteur, qui comprend un groupe provenant d'un groupe maléinimido, est produit par conversion du composé cytostatique avec un composé maléinimide de formule V, VI ou VII 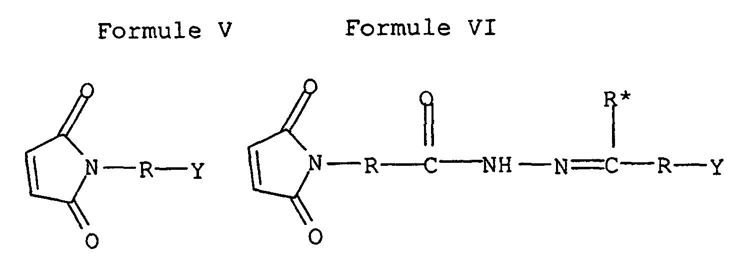 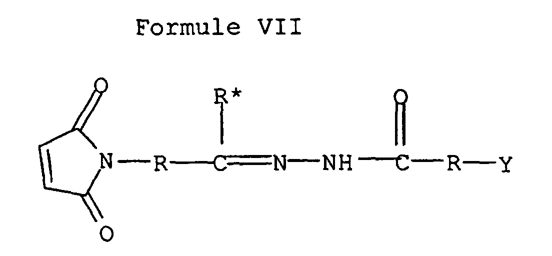 dans lesquelles, si R est une chaîne carbonée aliphatique ayant de 1 à 6 atomes de carbone, Y = -OH, -COOH, -COCl, -CONH- (CH₂) ₙ-OH, -COO- (CH₂) ₙ-NH₂, -COO-(CH₂)ₙ-NHNH₂, -SO₃H, -SO₃Cl, -SO₂-NHNH₂, -O-COCl, -CHO ou -COR* avec n = 1 - 6 et R* représente H, un groupe phényle ou alkyle ayant de 1 à 6 atomes de carbone et si R est un groupe benzyle substitué ou non substitué ou un groupe phényle substitué ou non substitué, Y = -OH, -COOH, -COCl, -CONH-(CH₂)ₙ-OH, -COO- (CH₂) ₙ-NH₂, -COO-(CH₂)ₙ-NHNH₂, -SO₃H, -SO₃Cl, -SO₂-NHNH₂, -O-COCl, -CHO ou -COR* avec n = 1 - 6 et R* représente H, un groupe phényle ou alkyle ayant de 1 à 6 atomes de carbone, et le dérivé de maléinimide ainsi obtenu du composé cytostatique est couplé à l'albumine thiolée, où la relation chimique entre le composé cytostatique et le composé maléinimide s'effectue par le biais d'une liaison amide, ester, imine, hydrazone, carboxylhydrazone, oxycarbonyle, acétal ou cétal.

5. Conjugué selon la revendication 3 ou 4, où le groupe provenant d'un composé bismaléinimide est produit par conjugaison de l'albumine thiolée couplée à 2 à 30 équivalents de composé cytostatique avec un composé bismaléinimide de formule XI 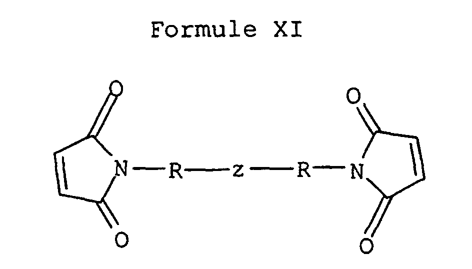 dans laquelle Z = -CO-NH-(CH₂)ₙ-NH-CO-, -CO-O-(CH₂)ₙ-O-CO-, -CH=N-(CH₂)ₙ-N=CH-, -CH=N-NH-(CH₂)ₙ-NH-N=CH- ou -CH=N-NH-CO-(CH₂)ₙ-CO-NH-N=CH- et n=2-12.

6. Procédé de préparation d'un conjugué d'un composé cytostatique et d'albumine selon l'une des revendications 1 à 5, comprenant les étapes de :
(a) conversion d'un composé cytostatique avec un composé maléinimide, de sorte que les dérivés maléinimide du composé cytostatique soient préparés, la relation chimique entre le composé cytostatique et le composé maléinimide s'effectuant par le biais d'une liaison amide, ester, imine, hydrazone, carboxylhydrazone, oxycarbonyle, acétal ou cétal, et
(b) chargement de l'albumine thiolée avec 2 à 30 équivalents de dérivé maléinimide, obtenu à l'étape (a), du composé cytostatique et conjugaison avec la transferrine ou un anticorps monoclonal dirigé contre un antigène associé aux tumeurs, par le biais d'un groupe provenant d'un composé bismaléinimide, l'albumine thiolée présentant en moyenne 1 à 30 groupes HS.

7. Médicament contenant un conjugué, selon l'une quelconque des revendications 1 à 5, éventuellement avec des supports et auxiliaires usuels.

8. Utilisation d'un conjugué, selon l'une quelconque des revendications 1 à 5, pour la préparation d'un médicament pour le traitement des maladies cancéreuses.

9. Utilisation selon la revendication 8, les maladies cancéreuses comprenant les carcinomes de la vésicule, des poumons, du sein, du mélanome et de la prostate.
